# EUROPEAN PATENT APPLICATION

(11) **EP 4 005 591 A1**
(43) Date of publication of application: **01.06.2022**
(21) Application number: 20843839.0
(22) Date of filing: 29.06.2020
(51) Int. Cl.: A61K 39/015, A61K 45/06, A61P 35/00, A61P 37/04

(54) **ANTI-NEOPLASTIC COMBINED PHARMACEUTICAL COMPOSITION AND APPLICATION THEREOF**

(30) Priority: 25.07.2019 CN 201910676072
(71) Applicant: Guangzhou Cas Lamvac Biotech Co., Ltd., Guangzhou, Guangdong 510530 (CN)
(72) Inventor: TAO, Zhu, Guangzhou, Guangdong 510530 (CN); KANG, Zhongkui, Guangzhou, Guangdong 510530 (CN); QIN, Li, Guangzhou, Guangdong 510530 (CN); CHEN, Xiaoping, Guangzhou, Guangdong 510530 (CN)
(74) Representative: HGF
(86) International application number: PCT/CN2020/098692
(87) International publication number: WO 2021/012886

(57) **Abstract**

An anti-neoplastic combined pharmaceutical composition and application thereof. The combined pharmaceutical composition is prepared from plasmodia and chemotherapeutic drugs. The combined pharmaceutical composition combines chemotherapy with plasmodium immunotherapy, has high biosafety, has stronger antineoplastic activity than single chemotherapy or single plasmodium immunotherapy, can prolong the lifetime of cancer patients, and provides a new strategy and idea for cancer treatment. Moreover, the dosage of the chemotherapy drugs can be reduced, toxic and side effects caused by the chemotherapy drugs are reduced, and treatment costs of tumor patients are reduced. In addition, the combined pharmaceutical composition can promote release of a tumor antigen, induces a stronger anti-tumor specific immune response, and exerts the continuous synergistic effect of immunotherapy and chemotherapy.

## Description

### TECHNICAL FIELD

The present application belongs to the field of biomedicine and relates to an anti-tumor combined pharmaceutical composition and use thereof.

### BACKGROUND

Surgery, radiotherapy and chemotherapy (including targeted therapy) are three traditional modes for treating tumors. A new mode is biotherapy (including immunotherapy). In clinics, combined therapy is mainly used. The combination of surgery with chemoradiotherapy has relatively good effects for treating early-stage tumors but poor effects for treating advanced tumors. At present, various biotherapies offer more options for the treatment of advanced malignant solid tumors. Targeted drugs, antibodies, immunotherapy drugs (such as PD-1) and cell therapy (such as CAR-T and TIL cells) have gained some progress but still cannot prevent the rapid development of cancer.

Malaria is an insect-borne disease caused by *Plasmodium* infection and transmitted through bites of *Anopheles* mosquitoes. Malaria is listed by World Health Organization as one of the three worldwide infectious diseases (AIDS, tuberculosis and malaria). *Plasmodium* for human infection mainly includes five types: *Plasmodium falciparum, Plasmodium vivax, Plasmodium malariae, Plasmodium ovale* and *Plasmodium knowlesi,* where *Plasmodium falciparum* and *Plasmodium vivax* are the most common ones. *Plasmodium* in mice mainly includes four types: *Plasmodium chabaudi, Plasmodium yoelii, Plasmodium berghei* and *Plasmodium vinckei.* It is proved in mice that *Plasmodium* infection significantly inhibits the tumor growth and metastasis and significantly prolongs the survival time of tumor-bearing mice in tumor models such as lung cancer, liver cancer, colon cancer and breast cancer.

In the clinical treatment of tumor, chemotherapy mainly kills tumor cells by cytotoxic drugs and cannot recognize tumor cells specifically, and the drugs have toxic and side effects. It is increasingly difficult to control tumor development with existing chemotherapy. The combined therapy of chemotherapy and biotherapy is a promising strategy for the treatment of tumor and comprehensively utilizes chemotherapy and biotherapy to exert the synergistic effect. Related reports exist in the existing art.

CN107929231A has disclosed an in situ thermosensitive gel drug delivery system for a combined chemoimmunotherapy. The delivery system is composed of a drug-loaded peptide dendrimer, an anti-tumor drug and a thermosensitive gel matrix material (e.g., PLGA-PEG-PLGA). The anti-tumor drug is entrapped in a cavity of a peptide dendrimer to form the drug-loaded peptide dendrimer and the drug-loaded peptide dendrimer is entrapped in a thermosensitive gel matrix through a swelling process to form the in-situ drug delivery system that is an injectable sol at room temperature and transformed into a gel at body temperature. The in-situ delivery system can form the gel near tumor and slowly release a drug-loaded peptide dendrimer molecule to act on tumor tissues, where the peptide dendrimer molecule acts on macrophages in the tumor tissues to generate NO which acts on the tumor together with the anti-tumor drug so as to practice the combined chemoimmunotherapy and inhibit the tumor development.

CN103347521A has disclosed a method for treating cancer, which includes administering a metabolically targeted chemotherapy-immunotherapy regimen. In one embodiment, the metabolically targeted chemotherapy-immunotherapy regimen may include the steps of administering a subject suffering from cancer with a therapeutically effective dosage of one or more immune agents (e.g., therapeutic antibodies) to stimulate an immune response and administering a therapeutically effective dosage of one or more chemotherapeutic agents. The method can be used for treating any type of cancer, especially malignant and transferred advanced cancer.

CN102481365A has disclosed a method for treating various types of cancer/tumor by administering a combination of a dll4 antagonist and a chemotherapeutic agent, where the dll4 antagonist is particularly a dll4 antibody and a fragment thereof that specifically binds to dll4. Such combined therapy exhibits the synergistic effect compared with the use of any therapeutic agent alone. Thus, the method is particularly beneficial for cancer patients with low tolerance to side effects caused by a desired high dosage of any therapeutic agent alone since the method can reduce an effective dosage. A pharmaceutical composition and a kit containing the dll4 antagonist and the chemotherapeutic agent are further provided.

In summary, there are a very limited number of strategies that combine chemotherapy with biotherapy for treating tumor and can achieve significant anti-tumor effects in the existing art. Therefore, it is very significant to develop a new biological chemotherapy strategy with a significant anti-tumor effect, which can provide a new cancer treatment idea.

### SUMMARY

The present application provides an anti-tumor combined pharmaceutical composition and a use thereof. The combined pharmaceutical composition successfully combines chemotherapy with *Plasmodium* immunotherapy (biotherapy), has relatively high biological safety, has stronger anti-tumor activity than single chemotherapy or single *Plasmodium* immunotherapy, can prolong the survival time of a tumor mouse model, and provides a new strategy and idea for the treatment of cancer. Moreover, the combined pharmaceutical composition can reduce a usage dosage of a chemotherapeutic agent, reduce the toxic and side effects of the chemotherapeutic agent, and reduce the cost at which tumor patients are treated. Additionally, the chemotherapeutic agent blocks the proliferation of tumor cells or directly kill tumor cells to cause the death of tumor cells, promote tumor cells to be phagocytized and processed by macrophages so as to release tumor antigens, and promote macrophages and dendritic cells to have a stronger antigen presentation ability under the induction of the *Plasmodium* immunotherapy, so as to induce a stronger anti-tumor specific immune response and exert a continuous synergistic effect of immunotherapy and chemotherapy.

In one aspect, the present application provides an anti-tumor combined pharmaceutical composition. The combined pharmaceutical composition includes *Plasmodium* and a chemotherapeutic agent.

*Plasmodium* in the combined pharmaceutical composition exerts an anti-tumor effect mainly by activating innate immunity and inducing certain specific immunity. Specifically, on one hand, dangerous signaling molecules released through *Plasmodium* infection, including a glycosylphosphatidylinositol-anchor, a heme, an immunostimulatory nucleic acid motif and other unknown molecules which are pathogen-associated pattern recognition molecules, can be recognized by pattern recognition receptors of immune cells of a host. The pattern recognition receptors activated by the pattern recognition molecules of *Plasmodium* trigger different transcription programs and stimulate multiple downstream signaling pathways to induce systemic immune responses which include releasing proinflammatory factors and Th1 cytokines, activating NK cells, NKT cells, macrophages and dendritic cells, further activating CD4⁺ and CD8⁺ T cells, inhibiting the generation of cytokines such as TGF-β and IL-10, and inhibiting the cell activity of regulatory T cells (Tregs), myeloid-derived suppressor cells (MDSCs) and tumor-associated macrophages (TAMs), so as to improve a tumor immunosuppressive microenvironment, transform an intratumoral immunosuppressive microenvironment in tumor patients into an immune activation state, and thereby, transform tumor into an effective tumor vaccine. On the other hand, *Plasmodium* infection activates immunity through damage-associated molecular patterns, and *Plasmodium-infected* red blood cells and known endogenous uric acid and microvesicles can all induce similar immunoactivity.

There are a wide variety of chemotherapeutic agents. According to their sources and chemical structures, the chemotherapeutic agents mainly include an alkylating agent-based chemotherapeutic agent, an anti-metabolic chemotherapeutic agent, an antibiotic-based chemotherapeutic agent, a hormone-based chemotherapeutic agent, an animal or plant chemotherapeutic agent and a miscellaneous chemotherapeutic agent. The alkylating agent-based chemotherapeutic agent directly acts on DNA to prevent the regeneration of cancer cells. Common alkylating agent-based chemotherapeutic agents include cyclophosphamide, ifosfamide and thiotepa. The anti-metabolic chemotherapeutic agent inhibits cell division and proliferation by interfering with the synthesis of DNAs and RNAs and mainly includes gemcitabine, pemetrexed, fluorouracil, cytarabine, methotrexate, mercaptopurine, hydroxyurea or the like. The antibiotic-based chemotherapeutic agent interferes with DNAs by inhibiting the action of enzymes and mitosis or changing cell membrane. The antibiotic-based chemotherapeutic agent is mainly a cell cycle non-specific agent and mainly includes mitomycin, daunorubicin, pingyangmycin, doxorubicin, actinomycin D, mitoxantrone or the like. Animal or plant chemotherapeutic agents are mainly plant bases and natural products, can inhibit mitosis or the action of enzymes to prevent the synthesis of essential proteins for cell regeneration, and mainly include vincristine, etoposide, teniposide, paclitaxel and docetaxel, which are commonly used in combination with other types of chemotherapeutic agents for the treatment of tumor. The hormone-based chemotherapeutic agent kills or slows down the growth of hormone-dependent tumor cells by using some hormones or antagonists and mainly includes tamoxifen, megestrol, triptorelin or medroxyprogesterone. The miscellaneous chemotherapeutic agent mainly includes cis-platinum, carboplatin, oxaliplatin, asparaginase or the like.

HIV protease inhibitors (HIV PIs) are a type of chemotherapeutic agents for treating HIV infection. They prevent aspartyl protein precursors from being cleaved into functional forms thereof by inhibiting the protease activity of viruses to prevent the maturation of HIV virus particles. HIV PIs mainly include nelfinavir, saquinavir, indinavir or ritonavir. Gills et al. have found that HIV PIs have anticancer activity against more than 60 cell lines and elucidated mechanisms including endoplasmic reticulum stress, autophagy, apoptosis and inhibition of Akt pathway. In patients with pancreatic cancer, nelfinavir in combination with gemcitabine, cis-platinum or radiotherapy can enhance an Akt inhibitory level and enhance sensitivity to radiotherapy, and no toxic and side effects from nelfinavir are found. Rengan et al. have found no nelfinavir dosage-dependent toxicity when nelfinavir is used in combination with etoposide, cis-platinum or radiotherapy for patients from who non-small cell lung cancer cannot be surgically exercised.

*Plasmodium* infection can systematically activate body's innate immunity and enhance body's specific immunity against tumor. The chemotherapeutic agents, including HIV PIs, can directly kill tumor cells or inhibit the proliferation of tumor cells. *Plasmodium* infection (*Plasmodium* immunotherapy) is combined with the chemotherapeutic agents to activate immunity and reduce tumor loads so as to exert a synergistic tumor treatment effect and has a more significant therapeutic effect than single *Plasmodium* immunotherapy or single chemotherapy.

In summary, the combined pharmaceutical composition successfully combines chemotherapy with *Plasmodium* immunotherapy (biotherapy), has relatively high biological safety, has stronger anti-tumor activity than single chemotherapy and single *Plasmodium* immunotherapy, can prolong the survival time of cancer mice, and provides a new strategy and idea for the treatment of cancer. Moreover, the combined pharmaceutical composition can reduce a usage dosage of the chemotherapeutic agent, reduce the toxic and side effects of the chemotherapeutic agent, and reduce the cost at which tumor patients are treated. Additionally, the combined pharmaceutical composition can promote tumor cells to release tumor antigens, induce a stronger anti-tumor specific immune response, and exert a continuous synergistic effect of immunotherapy and chemotherapy.

In the present application, the chemotherapeutic agent includes the alkylating agent-based chemotherapeutic agent, the anti-metabolic chemotherapeutic agent, the antibiotic-based chemotherapeutic agent, the animal or plant chemotherapeutic agent, the miscellaneous chemotherapeutic agent or the HIV protease inhibitor.

In a preferred embodiment, the alkylating agent-based chemotherapeutic agent includes cyclophosphamide or ifosfamide.

In a preferred embodiment, the anti-metabolic chemotherapeutic agent includes gemcitabine, pemetrexed, 5-fluorouracil, cytarabine or methotrexate.

In a preferred embodiment, the antibiotic-based chemotherapeutic agent includes mitomycin, doxorubicin or actinomycin D.

In a preferred embodiment, the animal or plant chemotherapeutic agent includes etoposide, docetaxel, paclitaxel, vincristine or irinotecan.

In a preferred embodiment, the miscellaneous chemotherapeutic agent includes cis-platinum, carboplatin, oxaliplatin or asparaginase.

In a preferred embodiment, the HIV protease inhibitor chemotherapeutic agent includes nelfinavir, saquinavir, indinavir or ritonavir.

In the present application, the combined pharmaceutical composition is in a dosage form that includes any pharmaceutically acceptable dosage form, such as tablets, powders, suspensions, granules, capsules, injections, sprays, solutions, enemas, emulsions, films, suppositories, patches, nasal drops or drop pills.

In a preferred embodiment, the combined pharmaceutical composition further includes any one or a combination of at least two of pharmaceutically acceptable adjuvants.

The combined pharmaceutical composition in the present application may be administered alone or combined with an adjuvant to form an appropriate dosage form for administration. The adjuvant includes any one or a combination of at least two of a diluent, an excipient, a filler, a binder, a wetting agent, a disintegrant, an emulsifier, a cosolvent, a solubilizer, an osmotic pressure regulator, a surfactant, a pH regulator, an antioxidant, a bacteriostatic agent or a buffer. The combination of at least two of the above adjuvants is, for example, a combination of the diluent and the excipient, a combination of the emulsifier and the cosolvent, a combination of the filler, the binder and the wetting agent or the like.

In a preferred embodiment, the combined pharmaceutical composition is a single compound preparation.

In another preferred embodiment, the combined pharmaceutical composition is a combination of two separate preparations.

In one embodiment, the two separate preparations are administered simultaneously.

In one embodiment, the two separate preparations are administered sequentially.

The combined pharmaceutical composition may be a single compound preparation or a combination of two separate preparations. When the combined pharmaceutical composition is a combination of two separate preparations, the combined pharmaceutical composition may be administered simultaneously or sequentially. For example, *Plasmodium* may be administered first, followed by the administration of the chemotherapeutic agent after a period of time, or the chemotherapeutic agent may be administered first, followed by the administration of *Plasmodium* after a period of time, or *Plasmodium* and the chemotherapeutic agent are administered alternately.

In the present application, the combined pharmaceutical composition is administered by a route that includes intravenous injection, intraperitoneal injection, intramuscular injection, subcutaneous injection, oral administration, sublingual administration, nasal administration or percutaneous administration, preferably intraperitoneal injection.

In a preferred embodiment, the combined pharmaceutical composition is loaded on a pharmaceutical carrier.

In one embodiment, the pharmaceutical carrier includes a liposome, a micelle, a dendrimer, a microsphere or a microcapsule.

In another aspect, the present application provides use of the preceding combined pharmaceutical composition for preparing a medicament against tumor.

In a preferred embodiment, the tumor includes lung cancer, gastric cancer, colon cancer, liver cancer, breast cancer or pancreatic cancer.

In another aspect, the present application provides a new anti-tumor combined therapy which is a combined therapy of chemotherapy and *Plasmodium* therapy. The anti-tumor combined therapy combines chemotherapy with *Plasmodium* immunotherapy, has relatively high biological safety, has stronger anti-tumor activity than single chemotherapy or single *Plasmodium* immunotherapy, can more effectively prolong the survival time of tumor-bearing mice, and provides a new strategy and idea for the treatment of cancer. Moreover, the anti-tumor combined therapy can reduce a usage dosage of a chemotherapeutic agent, reduce the toxic and side effects of the chemotherapeutic agent, and reduce the cost at which tumor patients are treated. Additionally, the combined therapy can promote tumor cells to release tumor antigens, induce a stronger anti-tumor specific response, and exert a continuous synergistic effect of immunotherapy and chemotherapy.

In a preferred embodiment, the tumor includes lung cancer, gastric cancer, colon cancer, liver cancer, breast cancer or pancreatic cancer.

In one embodiment, the chemotherapeutic agent used in chemotherapy includes an alkylating agent-based chemotherapeutic agent, an anti-metabolic chemotherapeutic agent, an antibiotic-based chemotherapeutic agent, an animal or plant chemotherapeutic agent, a miscellaneous chemotherapeutic agent or a HIV protease inhibitor.

In a preferred embodiment, the alkylating agent-based chemotherapeutic agent includes cyclophosphamide or ifosfamide.

In a preferred embodiment, the anti-metabolic chemotherapeutic agent includes gemcitabine, pemetrexed, 5-fluorouracil, cytarabine or methotrexate.

In a preferred embodiment, the antibiotic-based chemotherapeutic agent includes mitomycin, doxorubicin or actinomycin D.

In a preferred embodiment, the animal or plant chemotherapeutic agent includes etoposide, docetaxel, paclitaxel, vincristine or irinotecan.

In a preferred embodiment, the miscellaneous chemotherapeutic agent includes cis-platinum, carboplatin, oxaliplatin, asparaginase or the like.

In a preferred embodiment, the HIV protease inhibitor chemotherapeutic agent includes nelfinavir, saquinavir, indinavir or ritonavir.

In a preferred embodiment, the chemotherapy is administered by a route that includes intravenous injection, intraperitoneal injection, intramuscular injection, subcutaneous injection, oral administration, sublingual administration, nasal administration or percutaneous administration, preferably intravenous injection or oral administration.

In a preferred embodiment, a route of administration of *Plasmodium* therapy includes intravenous injection.

Compared with the existing art, the present application has the beneficial effects below.

The anti-tumor combined pharmaceutical composition involved in the present application combines chemotherapy with *Plasmodium* immunotherapy (biotherapy), has relatively high biological safety, has the stronger anti-tumor activity than single chemotherapy and single *Plasmodium* immunotherapy, can more effectively prolong the survival time of cancer patients, and provides the new strategy and idea for the treatment of cancer. Moreover, the combined pharmaceutical composition can reduce the usage dosage of the chemotherapeutic agent, reduce the toxic and side effects of the chemotherapeutic agent, and reduce the cost at which tumor patients are treated. Additionally, the combined pharmaceutical composition can promote tumor cells to release tumor antigens, induce the stronger anti-tumor specific response, and exert the continuous synergistic effect of immunotherapy and chemotherapy.

### BRIEF DESCRIPTION OF DRAWINGS

FIG. 1 is a graph of tumor growth in Example 1;
FIG. 2 is a graph illustrating survival of tumor-bearing mice in Example 1;
FIG. 3 is a graph of a *Plasmodium* infection rate in Example 1;
FIG. 4 is a graph illustrating weight growth of tumor-bearing mice in Example 1;
FIG. 5 is a graph of tumor growth in Example 2;
FIG. 6 is a graph illustrating survival of tumor-bearing mice in Example 2;
FIG. 7 is a graph of a *Plasmodium* infection rate in Example 2;
FIG. 8 is a graph illustrating weight growth of tumor-bearing mice in Example 2;
FIG. 9 is a graph of tumor growth in Example 3;
FIG. 10 is a graph illustrating survival of tumor-bearing mice in Example 3;
FIG. 11 is a graph of a *Plasmodium* infection rate in Example 3;
FIG. 12 is a graph illustrating weight growth of tumor-bearing mice in Example 3;
FIG. 13 is a graph of tumor growth in Example 4;
FIG. 14 is a graph illustrating survival of tumor-bearing mice in Example 4;
FIG. 15 is a graph of a *Plasmodium* infection rate in Example 4;
FIG. 16 is a graph illustrating weight growth of tumor-bearing mice in Example 4;
FIG. 17 is a graph of tumor growth in Example 5;
FIG. 18 is a graph illustrating survival of tumor-bearing mice in Example 5;
FIG. 19 is a graph of a *Plasmodium* infection rate in Example 5;
FIG. 20 is a graph illustrating weight growth of tumor-bearing mice in Example 5;
FIG. 21 is a graph of tumor growth in Example 6;
FIG. 22 is a graph illustrating survival of tumor-bearing mice in Example 6;
FIG. 23 is a graph of a *Plasmodium* infection rate in Example 6;
FIG. 24 is a graph illustrating weight growth of tumor-bearing mice in Example 6;
FIG. 25 is a graph of tumor growth in Example 7;
FIG. 26 is a graph illustrating survival of tumor-bearing mice in Example 7;
FIG. 27 is a graph of a *Plasmodium* infection rate in Example 7;
FIG. 28 is a graph illustrating weight growth of tumor-bearing mice in Example 7;
FIG. 29 is a graph of tumor growth in Example 8;
FIG. 30 is a graph illustrating survival of tumor-bearing mice in Example 8;
FIG. 31 is a graph of a *Plasmodium* infection rate in Example 8;
FIG. 32 is a graph illustrating weight growth of tumor-bearing mice in Example 8;
FIG. 33 is a graph of tumor growth in Example 9;
FIG. 34 is a graph illustrating survival of tumor-bearing mice in Example 9;
FIG. 35 is a graph of a *Plasmodium* infection rate in Example 9;
FIG. 36 is a graph illustrating weight growth of tumor-bearing mice in Example 9;
FIG. 37 is a graph of tumor growth in Example 10;
FIG. 38 is a graph illustrating survival of tumor-bearing mice in Example 10;
FIG. 39 is a graph of a *Plasmodium* infection rate in Example 10;
FIG. 40 is a graph illustrating weight growth of tumor-bearing mice in Example 10;
FIG. 41 is a graph of tumor growth in Example 11;
FIG. 42 is a graph illustrating survival of tumor-bearing mice in Example 11;
FIG. 43 is a graph of a *Plasmodium* infection rate in Example 11;
FIG. 44 is a graph illustrating weight growth of tumor-bearing mice in Example 11;
FIG. 45 is a graph of tumor growth in Example 12;
FIG. 46 is a graph illustrating survival of tumor-bearing mice in Example 12;
FIG. 47 is a graph of a *Plasmodium* infection rate in Example 12; and
FIG. 48 is a graph illustrating weight growth of tumor-bearing mice in Example 12.

### DETAILED DESCRIPTION

Technical solutions of the present application are further described below through the specific examples. Those skilled in the art are to understand that examples described herein are merely used for a better understanding of the present application and are not to be construed as specific limitations to the present application.

### Example 1

**This example provides an anti-tumor combined pharmaceutical composition. The combined pharmaceutical composition includes *Plasmodium yoelii* and a chemotherapeutic agent gemcitabine. The effect of gemcitabine in combination with *Plasmodium yoelii* in treating lung cancer at different administration times was observed.**

Experimental materials and reagents required in this example include the following:
Animals: C57BL/6 mice, female, at an age of 6 to 8 weeks, from SHANGHAI SLAC LABORATORY ANIMAL CO. LTD or BEIJING VITAL RIVER LABORATORY ANIMAL CO., LTD.;
*Plasmodium:* mouse *Plasmodium yoelii* (*P. yoelii 17XNL,* MRA-593, Py), free of charge from Malaria Research and Reference Reagent Resource Center (MR4);
Chemotherapeutic agent: gemcitabine (abbreviated as GEM), purchased from Sigma-Aldrich;
Giemsa stain powder: purchased from Sigma-Aldrich.

### (1) An animal model was established by the following specific method:

(I) Cell revival: the mouse Lewis lung carcinoma (LLC) cell line was revived and incubated in an incubator with 5% CO₂ and a constant temperature of 37 °C;
(II) Cell expansion: cells were passaged every 2-3 days, and when the cells grew to 80% of the bottom of the petri dish, the cells were digested with a 0.25% trypsin-EDTA digestion solution and diluted and passaged at a ratio of 1:3;
(III) Single cell preparation and cell inoculation: cells at a logarithmic growth phase were digested with trypsin, washed three times with PBS, re-suspended in a serum-free 1640 medium, and subcutaneously inoculated in the right scapular region of mice, each injected with 0.1 mL of cell suspension with an inoculum dose of 5×10⁵ LLC cells/mouse (where C57BL/6 mice were inoculated);
(IV) Experimental grouping: the mice were randomly divided into six groups according to a size of tumor: a tumor control group (Con), a *Plasmodium yoelii* treatment group (Py), a gemcitabine treatment group administered on day 3 (GEM (d3)), a gemcitabine treatment group administered on day 6 (GEM (d6)), a gemcitabine combined treatment group administered on day 3 (Py+GEM (d3)) and a gemcitabine combined treatment group administered on day 6 (Py+GEM (d6)). There were 10 mice in each group and 60 mice in total.

### (2) Tumor-bearing mice were inoculated with Plasmodium by the following specific method:

*(I) Plasmodium* revival: the murine *Plasmodium* blood (1.0 mL/vial) frozen in a liquid nitrogen tank was quickly shaken in a 37 °C water bath to be mixed and melted, and the activity of *Plasmodium* was kept.
(II) *Plasmodium* inoculation: after being mixed uniformly, the blood was inoculated intraperitoneally into C57BL/6 mice with 0.2 mL/mouse and two mice were inoculated each time.
(III) Preparation of thin blood film and microscopic examination: about 1-1.5 µL of blood was collected from tails of mice and smeared on slides so that a long tongue-shaped thin blood film with a length of 2.5 cm was prepared and blow-dried with a blower. The blood film was infiltrated with methanol for 1 min, stained with 1×Giemsa stain for 30 min, rinsed with tap water and blow-dried. The infection rate of *Plasmodium* was observed by a 100× oil immersion objective. Changes of the infection rate of *Plasmodium* were observed.
(IV) Preparation of a *Plasmodium* solution: when the infection rate reached 3% to 10%, the red blood cells were counted first, 5 µL of blood was taken from tails and re-suspended in 995 µL of PBS, and the red blood cells were counted. The number of red blood cells infected with *Plasmodium* per mL was calculated. The EP tube was moistened with 0.2 mL of 3.8% sodium citrate anticoagulant, the blood was taken from eyeballs, the required concentration and total amount of *Plasmodium* to be inoculated were calculated, and a concentration of 2.5×10⁶/mL was prepared with PBS.
(V) Inoculation of tumor-bearing mice: 7 days after tumor was subcutaneously inoculated, each mouse was inoculated with 0.2 mL of *Plasmodium,* that is, 5×10⁵ *Plasmodium* parasites.

### (3) Gemcitabine was administered by the following specific method:

(I) Mode of administration: intraperitoneal injection;
(II) Dosage of administration: a total dosage of 100 mg/kg;
(III) Drug preparation: gemcitabine was dissolved in normal saline to be prepared into a solution with a concentration of 10 mg/mL;
(IV) Time of administration: in the GEM (d3) treatment group and the Py+GEM (d3) treatment group, drug was administered on day 3 after tumor inoculation, and in the GEM (d6) treatment group and the Py+GEM (d6) treatment group, drug was administered on day 6 after tumor inoculation.

### (4) Detection indicators

(I) Measurement of tumor volume: the volume of tumor was measured every three days by the formula for calculating the volume of an ellipsoid (unit: cubic mm): (D×d×d)/2, where D denotes a long diameter of tumor and d denotes a short diameter of tumor. The size of tumor was represented by a mean tumor volume ± the standard error of the mean (SEM) and a tumor growth curve was drawn. An intergroup statistical analysis was performed through a two-way analysis of variance (ANOVA), where "^{∗}" represents *p* ≤ 0.05 and "^{∗∗}" represents *p* ≤ 0.01, both indicating that differences between groups were statistically significant.
(II) Survival statistics of mice: the survival was evaluated by a median survival time and a percentage of a prolonged survival time. The survival rate was estimated by a Kaplan-Meier method, a survival curve was drawn, and the median survival time was calculated. "^{∗}" represents *p* ≤ 0.05 and "^{∗∗}" represents *p* ≤ 0.01, both indicating that differences between groups were statistically significant.
(III) Statistics of the infection rate of *Plasmodium:* the infection rate of *Plasmodium* was evaluated by a percentage of red blood cells infected with *Plasmodium* in mice, where the calculation formula was (the number of red blood cells infected with *Plasmodium*/*a* total number of red blood cells) × 100%. Specific procedures were as follows: blood was taken from tail veins and smeared on a slide, fixed with methanol, and stained with Giemsa stain. The number of red blood cells infected with *Plasmodium* and the total number of red blood cells were observed under a microscope. The total number of red blood cells was about 1000. The infection rate of *Plasmodium* was calculated and represented by a mean infection rate ± the standard error of the mean (SEM), a *Plasmodium* infection cycle curve was drawn, and whether the chemotherapeutic agent has an effect on *Plasmodium* infection was observed.
(IV) Weight of mice: The mice were weighed every three days and the weight growth was represented by a mean weight ± the standard error of the mean (SEM). The effects of the chemotherapeutic agent and *Plasmodium* infection on the weight of tumor-bearing mice were observed.

### (5) Experimental results

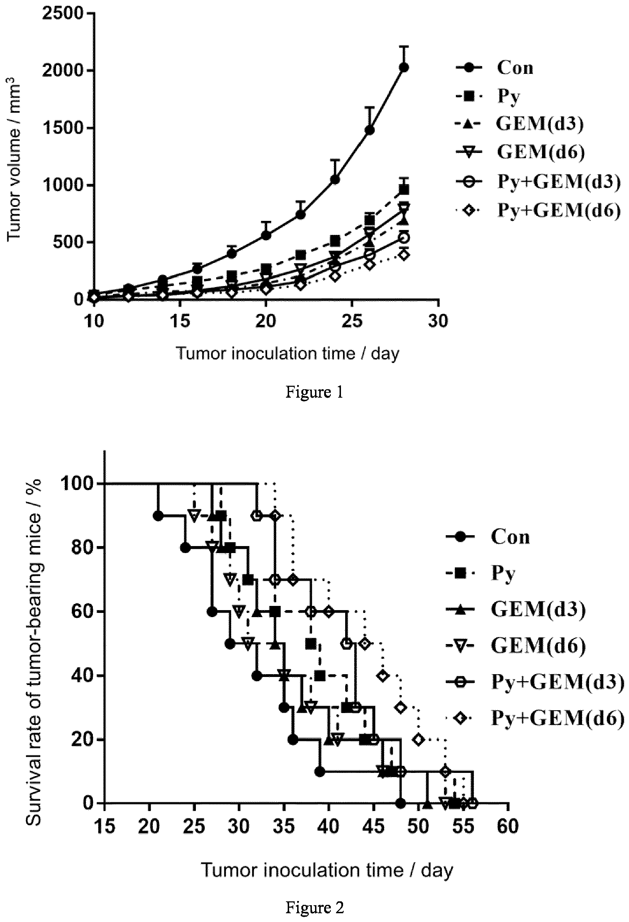

(I) As shown in FIG. 1 and Table 1, the *Plasmodium yoelii* treatment group (Py), the gemcitabine treatment group administered on day 3 (GEM (d3)), the gemcitabine treatment group administered on day 6 (GEM (d6)), the gemcitabine combined treatment group administered on day 3 (Py+GEM (d3)) and the gemcitabine combined treatment group administered on day 6 (Py+GEM (d6)) can all significantly inhibit the growth of lung cancer. The combined treatment group administered on day 6 may have a better effect in inhibiting lung cancer than the combined treatment group administered on day 3, without statistical significance. The combined treatment group administered on day 6 may have the better effect in inhibiting lung cancer than the single *Plasmodium yoelii* treatment group and the single gemcitabine treatment group administered on day 6, with statistical significance.

**Table 1**

| Intergroup Comparison | Significance |
|---|---|
| Con vs Py | ** |
| Con vs GEM (d3) | ** |
| Con vs GEM (d6) | ** |
| Con vs Py+GEM (d3) | ** |
| Con vs Py+GEM (d6) | ** |
| Py vs Py+GEM (d3) | ** |
| Py vs Py+GEM (d6) | ^{∗∗} |
| GEM (d6) vs Py+GEM (d6) | ^{∗∗} |

(II) As shown in FIG. 2 and Table 2, the median survival time of the tumor control group is 30.5 days, the median survival time of the *Plasmodium yoelii* treatment group is 38.5 days, the median survival time of the gemcitabine treatment group administered on day 3 is 34.5 days, the median survival time of the gemcitabine treatment group administered on day 6 is 33 days, the median survival time of the combined treatment group administered on day 3 is 42.5 days, and the median survival time of the combined treatment group administered on day 6 is 45 days. The *Plasmodium yoelii* treatment group (Py), the gemcitabine treatment group administered on day 3 (GEM (d3)), the gemcitabine treatment group administered on day 6 (GEM (d6)), the gemcitabine combined treatment group administered on day 3 (Py+GEM (d3)) and the gemcitabine combined treatment group administered on day 6 (Py+GEM (d6)) can all significantly prolong the survival time of tumor-bearing mice. The combined treatment group administered on day 6 has a longer median survival time than the combined treatment group administered on day 3, without statistical significance. The combined treatment with administration on day 6 has the longer median survival time than single *Plasmodium yoelii* treatment and single gemcitabine treatment, but survival differences have no statistical significance.

**Table 2**

| Intergroup Comparison | Significance |
|---|---|
| Con vs GEM (d3) | * |
| Con vs GEM (d6) | ** |
| GEM (d6) vs Py+GEM (d6) | * |

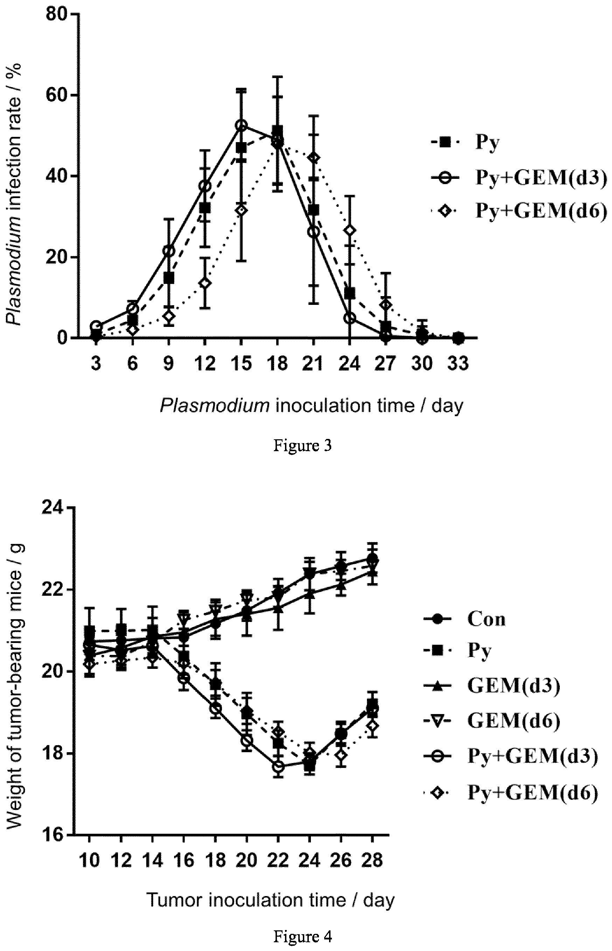

(III) As shown in FIG. 3, tumor-bearing mice in the *Plasmodium yoelii* treatment group and two combined treatment groups have a relatively consistent parasitemia duration period, where the infection period is about one month. The combined treatment group administered on day 3 has an earlier peak of *Plasmodium yoelii* infection, and the combined treatment group administered on day 6 has a later peak of *Plasmodium yoelii* infection, indicating that gemcitabine has a certain inhibitory effect on *Plasmodium yoelii* but will not eliminate *Plasmodium.*
(IV) As shown in FIG. 4, the weight of tumor-bearing mice in the combined treatment group is mainly affected by *Plasmodium* and affected little by gemcitabine, and gemcitabine in combination with *Plasmodium* has a synergistic effect in reducing the weight of tumor-bearing mice.

**(6) Summary:** The gemcitabine combined treatment group administered on day 6 can significantly inhibit the growth of lung cancer and prolong the median survival time of tumor-bearing mice. Gemcitabine has an inhibitory effect on *Plasmodium* but will not eliminate *Plasmodium.* Gemcitabine in combination with *Plasmodium* has a synergistic effect in reducing the weight of tumor-bearing mice. Gemcitabine administered on day 6 at a total dosage of 100 mg/kg and *Plasmodium yoelii* inoculated on day 7 are a potential combined pharmaceutical composition for the treatment of lung cancer.

### Example 2

### This example provides an anti-tumor combined pharmaceutical composition. The combined pharmaceutical composition includes Plasmodium yoelii and a chemotherapeutic agent gemcitabine. The effect of gemcitabine, administered in a single dose or divided doses at a same total dosage, in combination with Plasmodium yoelii therapy in treating lung cancer was observed.

The experimental materials and reagents used in this example were the same as those in Example 1.

### (1) An animal model was established by the following specific method:

Steps (I), (II) and (III) were the same as those in Example 1.

(IV) Experimental grouping: mice were randomly divided into six groups according to a size of tumor: a tumor control group (Con), a *Plasmodium yoelii* treatment group (Py), a single-dose gemcitabine treatment group (GEM (single)), a divided-dose gemcitabine treatment group (GEM (divided)), a single-dose combined treatment group (Py+GEM (single)) and a divided-dose combined treatment group (Py+GEM (divided)). There were 10 mice in each group and 60 mice in total.

### (2) Tumor-bearing mice were inoculated with Plasmodium by the following specific method:

Steps (I), (II), (III), (IV) and (V) were the same as those in Example 1.

### (3) Gemcitabine was administered by the following specific method:

(I) Mode of administration: intraperitoneal injection;
(II) Dosage of administration: a total dosage of 100 mg/kg;
(III) Drug preparation: gemcitabine was dissolved in normal saline to be prepared into a solution with a concentration of 5 mg/mL and a solution with a concentration of 10 mg/mL;
(IV) Time of administration: in the GEM (single) treatment group and Py+GEM (single) treatment group, drug was administered on day 6 after tumor inoculation, at a dosage of 100 mg/kg and with a concentration of 10 mg/mL; in the GEM (divided) treatment group and the Py+GEM (divided) treatment group, drug was administered on day 6 and day 13 after tumor inoculation, respectively, at a dosage of 50 mg/kg each time and with a concentration of 5 mg/kg.

### (4) Detection indicators were the same as those in Example 1.

### (5) Experimental results

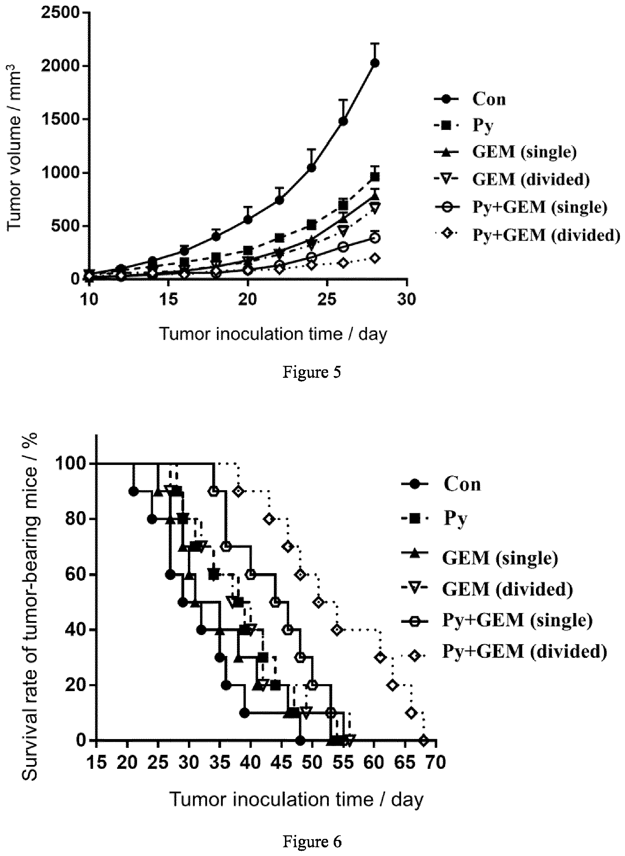

(I) As shown in FIG. 5 and Table 3, the tumor control group (Con), the *Plasmodium yoelii* treatment group (Py), the single-dose gemcitabine treatment group (GEM (single)), the divided-dose gemcitabine treatment group (GEM (divided)), the single-dose combined treatment group (Py+GEM (single)) and the divided-dose combined treatment group (Py+GEM (divided)) all significantly inhibit the growth of lung cancer. The divided-dose gemcitabine combined treatment group has a better effect in inhibiting the growth of lung cancer than the single-dose gemcitabine combined treatment group. Each of the two combined treatment groups has a better effect in inhibiting the growth of lung cancer than the single *Plasmodium yoelii* treatment group and the single gemcitabine treatment group.

**Table 3**

| Intergroup Comparison | Significance |
|---|---|
| Con vs Py | ** |
| Con vs GEM (single) | ** |
| Con vs GEM (divided) | ** |
| Con vs Py+ GEM (single) | ** |
| Con vs Py+ GEM (divided) | ** |
| Py vs Py+ GEM (single) | ** |
| Py vs Py+ GEM (divided) | ** |
| GEM (single) vs Py+ GEM (single) | ** |
| GEM (divided) vs Py+ GEM (divided) | ** |
| Py+ GEM (single) vs Py+ GEM (divided) | ** |

(II) As shown in FIG. 6 and Table 4, the median survival time of the tumor control group is 30.5 days, the median survival time of the *Plasmodium yoelii* treatment group is 38.5 days, the median survival time of the single-dose gemcitabine treatment group is 33 days, the median survival time of the gemcitabine treatment group with the divided administration is 38.5 days, the median survival time of the treatment group with a single dose of gemcitabine in combination with *Plasmodium yoelii* is 45 days, and the median survival time of the treatment group with a divided administration of gemcitabine in combination with *Plasmodium yoelii* is 52.5 days. The two combined treatment groups significantly prolong the median survival time of tumor-bearing mice. Each of the two combined treatment groups more effectively prolongs the median survival time of tumor-bearing mice than the corresponding single gemcitabine treatment group. The divided-dose combined treatment group more effectively prolongs the survival time of tumor-bearing mice than the single-dose combined treatment group. The survival difference between the single-dose combined therapy and the single *Plasmodium* therapy has no statistical significance, but the divided-dose combined therapy more effectively prolongs the median survival time of tumor-bearing mice than the single *Plasmodium* therapy.

**Table 4**

| Intergroup Comparison | Significance |
|---|---|
| Con vs Py+GEM (single) | * |
| Con vs Py+GEM (divided) | ** |
| Py vs Py+GEM (divided) | ** |
| GEM (single) vs Py+GEM (single) | * |
| GEM (divided) vs Py+GEM (divided) | ** |
| Py+GEM (single) vs Py+GEM (divided) | * |

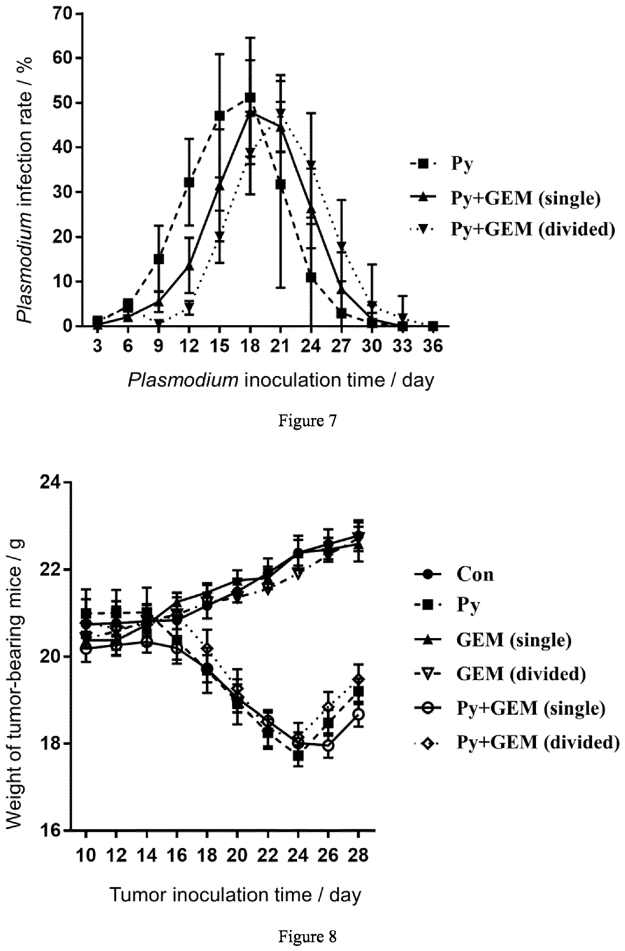

(III) As shown in FIG. 7, tumor-bearing mice in the *Plasmodium yoelii* treatment group and two gemcitabine combined treatment groups have a relatively consistent parasitemia duration period, where the infection period is about one month. The single dose of gemcitabine and divided doses of gemcitabine both delay the peak of *Plasmodium,* where the divided dose has a better delay effect. This indicates that gemcitabine has a certain inhibitory effect on *Plasmodium.*
(IV) As shown in FIG. 8, the weight of tumor-bearing mice in the combined treatment group is mainly affected by *Plasmodium* and affected little by gemcitabine, and gemcitabine in combination with *Plasmodium* has a slight synergistic effect in reducing the weight of tumor-bearing mice, but the divided doses have a smaller effect on the weight of tumor-bearing mice than the single dose.

**(6) Summary:** Divided-dose gemcitabine combined therapy has a better effect than single-dose gemcitabine combined therapy in inhibiting the growth of lung cancer and prolonging the median survival time of tumor-bearing mice. The single dose of gemcitabine and the divided doses of gemcitabine both inhibit *Plasmodium yoelii.* The divided doses of gemcitabine have the smaller effect on the weight of tumor-bearing mice than the single dose of gemcitabine. Gemcitabine administrated in divided doses on day 6 and day 13 at a total dosage of 100 mg/kg and *Plasmodium yoelii* inoculated on day 7 are a potential combined pharmaceutical composition for the treatment of lung cancer.

### Example 3

### This example provides an anti-tumor combined pharmaceutical composition. The combined pharmaceutical composition includes Plasmodium yoelii and a chemotherapeutic agent gemcitabine. The effect of an optimized regimen of gemcitabine administration in combination with Plasmodium immunotherapy in treating lung cancer was observed.

The experimental materials and reagents required in this example were the same as those in Example 1.

### (1) An animal model was established by the following specific method:

Steps (I), (II) and (III) were the same as those in Example 1.

(IV) Experimental grouping: mice were randomly divided into four groups according to a size of tumor: a tumor control group (Con), a *Plasmodium* treatment group (Py), a gemcitabine treatment group (GEM) and a treatment group of gemcitabine in combination with *Plasmodium* (Py+GEM). There were 15 mice in each group and 60 mice in total.

### (2) Tumor-bearing mice were inoculated with Plasmodium by the following specific method:

Steps (I), (II), (III), (IV) and (V) were the same as those in Example 1.

### (3) Gemcitabine was administered by the following specific method:

(I) Mode of administration: intraperitoneal injection;
(II) Dosage of administration: 100 mg/kg;
(III) Drug preparation: gemcitabine was dissolved in normal saline to be prepared into a solution with a concentration of 5 mg/mL;
(IV) Time of administration: in the GEM treatment group and the Py+GEM treatment group, drug was administered on day 6 and day 13 after tumor inoculation at a dosage of 50 mg/kg each time.

### (4) Detection indicators were the same as those in Example 1.

### (5) Experimental results

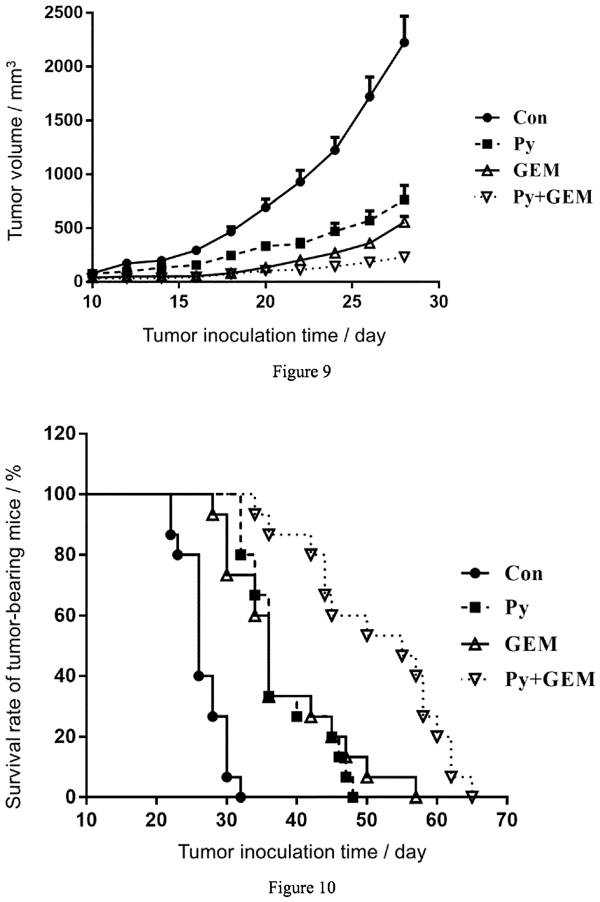

(I) As shown in FIG. 9 and Table 5, the tumor control group (Con), the *Plasmodium* treatment group (Py), the gemcitabine treatment group (GEM) and the treatment group of gemcitabine in combination with *Plasmodium* (Py+GEM) all significantly inhibit the growth of lung cancer. Combined therapy of gemcitabine with *Plasmodium* more effectively inhibits tumor growth than single gemcitabine therapy and single *Plasmodium yoelii* therapy. This indicates that a combined pharmaceutical composition of gemcitabine and *Plasmodium yoelii* can more effectively inhibit the growth of lung cancer.

**Table 5**

| Intergroup Comparison | Significance |
|---|---|
| Con vs Py | ** |
| Con vs GEM | ** |
| Con vs Py+GEM | ** |
| Py vs Py+GEM | ** |
| GEM vs Py+GEM | ** |

(II) As shown in FIG. 10 and Table 6, the median survival time of the tumor control group is 26 days, the median survival time of the *Plasmodium yoelii* treatment group is 36 days, the median survival time of the gemcitabine treatment group is 36 days, and the median survival time of the combined treatment group is 55 days. The combined therapy more effectively prolongs the survival of tumor-bearing mice than the single *Plasmodium yoelii* therapy and the single gemcitabine therapy. This indicates that the combined pharmaceutical composition of gemcitabine and *Plasmodium yoelii* can significantly prolong the survival of tumor-bearing mice.

**Table 6**

| Intergroup Comparison | Significance |
|---|---|
| Con vs Py | ** |
| Con vs GEM | ** |
| Con vs Py+GEM | ** |
| Py vs Py+GEM | ** |
| GEM vs Py+GEM | ** |

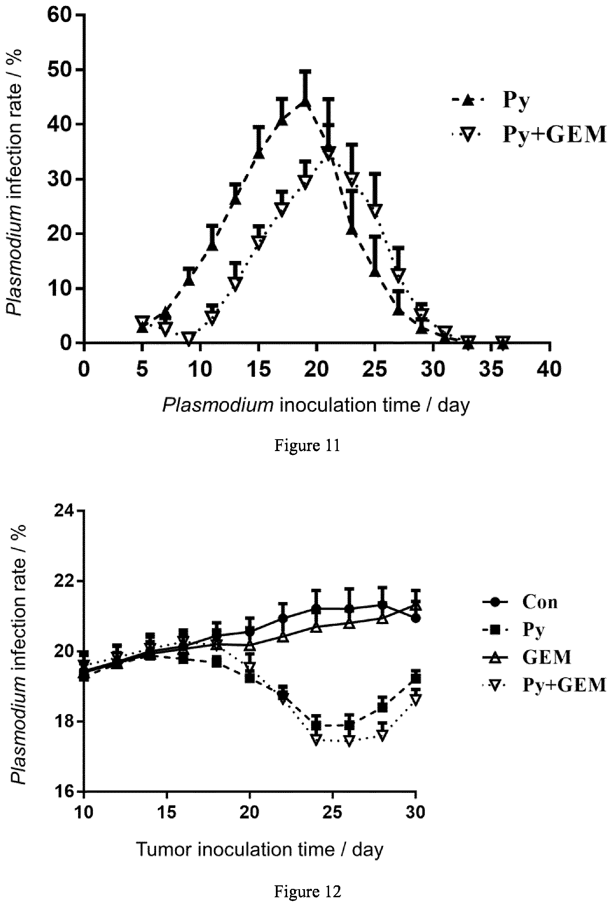

(III) As shown in FIG. 11, gemcitabine delays the peak of *Plasmodium* and a parasitemia duration period remains unchanged. Gemcitabine has a certain inhibitory effect on *Plasmodium yoelii* but will not eliminate *Plasmodium yoelii.* This indicates that gemcitabine can be combined with *Plasmodium yoelii* for the treatment of lung cancer, and two therapies have little interference.
(IV) As shown in FIG. 12, the weight of tumor-bearing mice in the combined treatment group is mainly affected by *Plasmodium* and affected little by gemcitabine, and gemcitabine in combination with *Plasmodium* has a slight synergistic effect in reducing the weight of tumor-bearing mice without significantly increasing toxic and side effects on tumor-bearing mice.

**(5) Summary:** The combined pharmaceutical composition of gemcitabine and *Plasmodium yoelii* can more effectively inhibit the growth of lung cancer and prolong the median survival time of tumor-bearing mice without increasing the toxic and side effects on tumor-bearing mice. Gemcitabine administrated in divided doses on day 6 and day 13 at a total dosage of 100 mg/kg and *Plasmodium yoelii* inoculated on day 7 are a potential combined pharmaceutical composition for the treatment of lung cancer.

### Example 4

### This example provides an anti-tumor combined pharmaceutical composition. The combined pharmaceutical composition includes Plasmodium chabaudi and a chemotherapeutic agent gemcitabine. The effect of an optimized regimen of gemcitabine administration in combination with Plasmodium chabaudi immunotherapy in treating lung cancer was observed.

Experimental materials and reagents required in this example differed from those in Example 3 only in that mouse *Plasmodium chabaudi* (*Plasmodium chabaudi,* MRA-429, Pc) was used, which was free of charge from Malaria Research and Reference Reagent Resource Center (MR4).

### (1) An animal model was established by the following specific method:

Steps (I), (II) and (III) were the same as those in Example 3.

(IV) Experimental grouping: mice were randomly divided into four groups according to a size of tumor: a tumor control group (LLC), a *Plasmodium chabaudi* treatment group (Pc), a gemcitabine treatment group (GEM) and a treatment group of gemcitabine in combination with *Plasmodium chabaudi* (Pc+GEM). There were 10 mice in each group and 40 mice in total.

### (2) Tumor-bearing mice were inoculated with Plasmodium by the same specific method as in Example 3.

### (3) Gemcitabine was administered by the following specific method:

(I) Mode of administration: intraperitoneal injection;
(II) Dosage of administration: a total dosage of 100 mg/kg;
(III) Drug preparation: gemcitabine was dissolved in normal saline to be prepared into a solution with a concentration of 5 mg/mL;
(IV) Time of administration: in the GEM treatment group and the Pc+GEM treatment group, drug was administered on day 6 and day 13 after tumor inoculation at a dosage of 50 mg/kg each time.

### (4) Detection indicators were the same as those in Example 3.

### (5) Experimental results

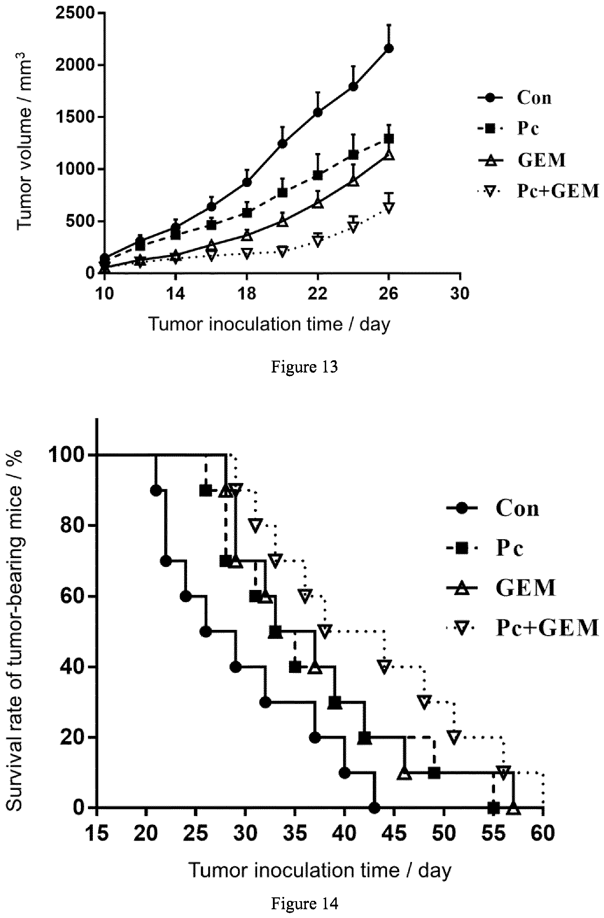

(I) As shown in FIG. 13 and Table 7, the tumor control group (LLC), the *Plasmodium chabaudi* treatment group (Pc), the gemcitabine treatment group (GEM) and the treatment group of gemcitabine in combination with *Plasmodium chabaudi* (Pc+GEM) all significantly inhibit the growth of lung cancer. Combined therapy more effectively inhibits tumor growth than the single *Plasmodium chabaudi* treatment group and the single gemcitabine treatment group. This indicates that a combined pharmaceutical composition of gemcitabine and *Plasmodium chabaudi* can more effectively inhibit the growth of lung cancer.

**Table 7**

| Intergroup Comparison | Significance |
|---|---|
| Con vs Pc | ** |
| Con vs GEM | ** |
| Con vs Pc+ GEM | ** |
| Pc vs Pc+ GEM | * |
| GEM vs Pc+ GEM | ** |

(II) As shown in FIG. 14 and Table 8, the median survival time of tumor-bearing mice is compared: the median survival time of the tumor group is 27.5 days, the median survival time of the *Plasmodium chabaudi* treatment group is 34 days, the median survival time of the gemcitabine treatment group is 35 days, and the median survival time of the combined treatment group is 41 days. The combined therapy significantly prolongs the survival of tumor-bearing mice, but survival differences of the combined therapy from single *Plasmodium chabaudi* therapy and single gemcitabine therapy have no statistical significance. This indicates that the combined pharmaceutical composition of gemcitabine and *Plasmodium chabaudi* can significantly prolong the survival of tumor-bearing mice.

**Table 8**

| Intergroup Comparison | Significance |
|---|---|
| Con vs Pc+GEM | ** |

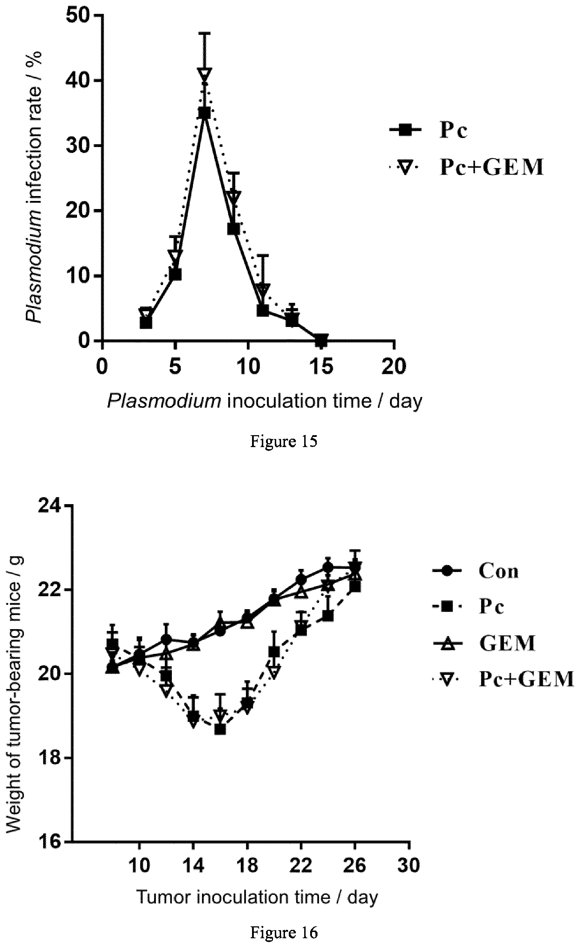

(III) As shown in FIG. 15, tumor-bearing mice in the *Plasmodium chabaudi* treatment group and the combined treatment group have a consistent parasitemia duration period. Gemcitabine has an inhibitory effect on *Plasmodium chabaudi* but will not eliminate *Plasmodium.* This indicates that gemcitabine can be combined with *Plasmodium chabaudi* for the treatment of lung cancer, and two therapies have little interference.
(IV) As shown in FIG. 16, the weight of tumor-bearing mice in the combined treatment group is mainly affected by *Plasmodium chabaudi* and affected little by gemcitabine, and gemcitabine in combination with *Plasmodium* has a slight synergistic effect in reducing the weight of tumor-bearing mice without significantly increasing toxic and side effects on tumor-bearing mice.

**(6) Summary:** The combined pharmaceutical composition of gemcitabine and *Plasmodium chabaudi* can more effectively inhibit the growth of lung cancer and prolong the median survival time of tumor-bearing mice without increasing the toxic and side effects on tumor-bearing mice. Gemcitabine administrated in divided doses on day 6 and day 13 at a total dosage of 100 mg/kg and *Plasmodium chabaudi* inoculated on day 7 are a potential combined pharmaceutical composition for the treatment of lung cancer.

### Example 5

### This example provides an anti-tumor combined pharmaceutical composition. The combined pharmaceutical composition includes Plasmodium yoelii and a chemotherapeutic agent cyclophosphamide. The effect of cyclophosphamide administration in combination with Plasmodium yoelii immunotherapy in treating lung cancer was observed.

Experimental materials and reagents required in this example differed from those in Example 1 only in that the chemotherapeutic agent was cyclophosphamide (abbreviated as CTX), which was purchased from Sigma-Aldrich.

### (1) An animal model was established by the following specific method:

Steps (I), (II) and (III) were the same as those in Example 3.

(IV) Experimental grouping: mice were randomly divided into four groups according to a size of tumor: a tumor control group (Con), a *Plasmodium* treatment group (Py), a cyclophosphamide treatment group (CTX) and a treatment group of cyclophosphamide in combination with *Plasmodium* (Py +CTX). There were 11 mice in each group and 44 mice in total.

### (2) Tumor-bearing mice were inoculated with Plasmodium by the same specific method as in Example 3.

### (3) Cyclophosphamide was administered by the following specific method:

(I) Mode of administration: intraperitoneal injection;
(II) Dosage of administration: 20 mg/kg×3;
(III) Drug preparation: cyclophosphamide was dissolved in normal saline to be prepared into a solution with a concentration of 2 mg/mL;
(IV) Time of administration: in the CTX treatment group and the Py+CTX treatment group, drug was administered on day 6, day 13 and day 20 after tumor inoculation at a dosage of 20 mg/kg each time.

### (4) Detection indicators were the same as those in Example 3.

### (5) Experimental results

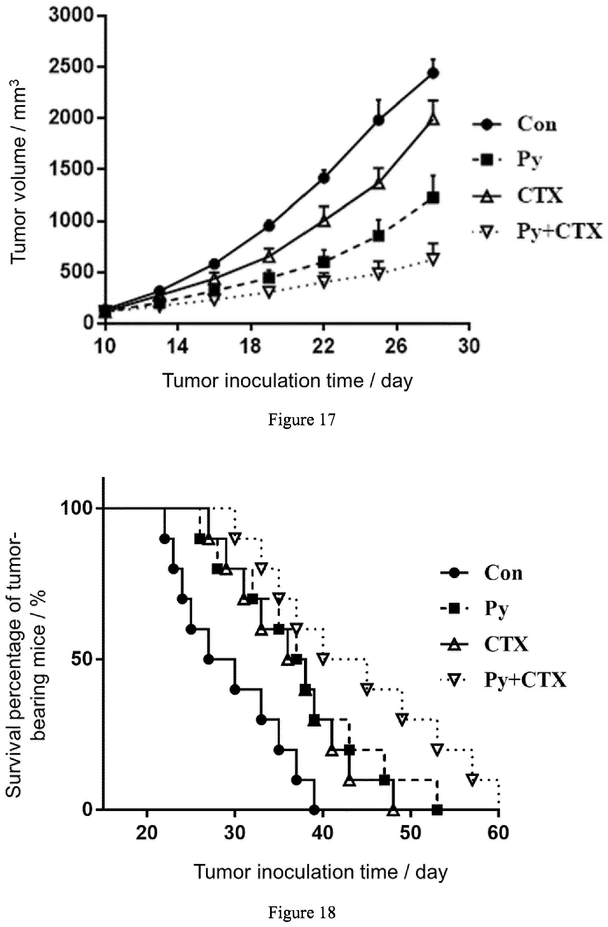

(I) As shown in FIG. 17 and Table 9, the tumor control group (Con), the *Plasmodium* treatment group (Py), the cyclophosphamide treatment group (CTX) and the treatment group of cyclophosphamide in combination with *Plasmodium* (Py+CTX) all significantly inhibit the growth of lung cancer. Combined therapy more effectively inhibits tumor growth than the single cyclophosphamide treatment group and the single *Plasmodium yoelii* treatment group. This indicates that a combined pharmaceutical composition of cyclophosphamide and *Plasmodium yoelii* can more effectively inhibit the growth of lung cancer.

**Table 9**

| Intergroup Comparison | Significance |
|---|---|
| Con vs Py | ** |
| Con vs CTX | * |
| Con vs Py+CTX | ** |
| Py vs Py+ CTX | * |
| CTX vs Py+CTX | ** |

(II) As shown in FIG. 18 and Table 10, the median survival time of tumor-bearing mice is compared: the median survival time of the tumor group is 28.5 days, the median survival time of the *Plasmodium yoelii* treatment group is 37.5 days, the median survival time of the cyclophosphamide treatment group is 37 days, and the median survival time of the combined treatment group is 42.5 days. The single *Plasmodium* treatment group, the single cyclophosphamide treatment group and the combined treatment group all significantly prolong the survival of tumor-bearing mice. Survival differences of the combined therapy from the single *Plasmodium* treatment group and the single cyclophosphamide treatment group have no statistical significance, which still suggests that the combined pharmaceutical composition of cyclophosphamide and *Plasmodium yoelii* may prolong the survival of tumor-bearing mice.

**Table 10**

| Intergroup Comparison | Significance |
|---|---|
| Con vs Py | * |
| Con vs CTX | ** |
| Con vs Py+CTX | ** |

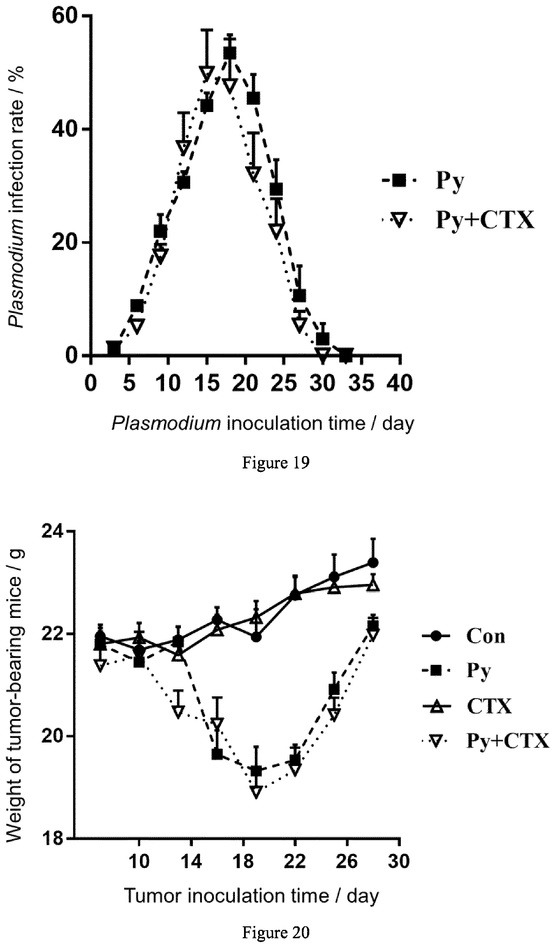

(III) As shown in FIG. 19, cyclophosphamide has a relatively small effect on *Plasmodium,* and tumor-bearing mice in the *Plasmodium yoelii* treatment group and the combined treatment group have a relatively consistent parasitemia infection duration period. This indicates that cyclophosphamide can be combined with *Plasmodium yoelii* for the treatment of lung cancer, and two therapies have little interference.
(IV) As shown in FIG. 20, the weight of tumor-bearing mice in the combined treatment group is mainly affected by *Plasmodium chabaudi* and affected little by cyclophosphamide, and cyclophosphamide in combination with *Plasmodium* has a slight synergistic effect in reducing the weight of tumor-bearing mice without significantly increasing toxic and side effects on tumor-bearing mice.

**(5) Summary:** The combined pharmaceutical composition of cyclophosphamide and *Plasmodium yoelii* can more effectively inhibit the growth of lung cancer and prolong the median survival time of tumor-bearing mice without significantly increasing the toxic and side effects on tumor-bearing mice. Cyclophosphamide administrated in three divided doses on day 6, day 13 and day 20 at a total dosage of 60 mg/kg and *Plasmodium yoelii* inoculated on day 7 are a potential combined pharmaceutical composition for the treatment of lung cancer.

### Example 6

### This example provides an anti-tumor combined pharmaceutical composition. The combined pharmaceutical composition includes Plasmodium chabaudi and a chemotherapeutic agent cyclophosphamide. The effect of an optimized regimen of cyclophosphamide administration in combination with Plasmodium chabaudi therapy in treating lung cancer was observed.

Experimental materials and reagents required in this example differed from those in Example 5 only in that mouse *Plasmodium chabaudi* (*Plasmodium chabaudi,* MRA-429, Pc) was used, which was free of charge from Malaria Research and Reference Reagent Resource Center (MR4).

### (1) An animal model was established by the following specific method:

Steps (I), (II) and (III) were the same as those in Example 5.

(IV) Experimental grouping: mice were randomly divided into four groups according to a size of tumor: a tumor control group (Con), a *Plasmodium* treatment group (Pc), a cyclophosphamide treatment group (CTX) and a treatment group of cyclophosphamide in combination with *Plasmodium* (Pc +CTX). There were 10 mice in each group and 40 mice in total.

### (2) Tumor-bearing mice were inoculated with Plasmodium by the same specific method as in Example 5.

### (3) Cyclophosphamide was administered by the same specific method as in Example 5.

### (4) Detection indicators were the same as those in Example 5.

### (5) Experimental results

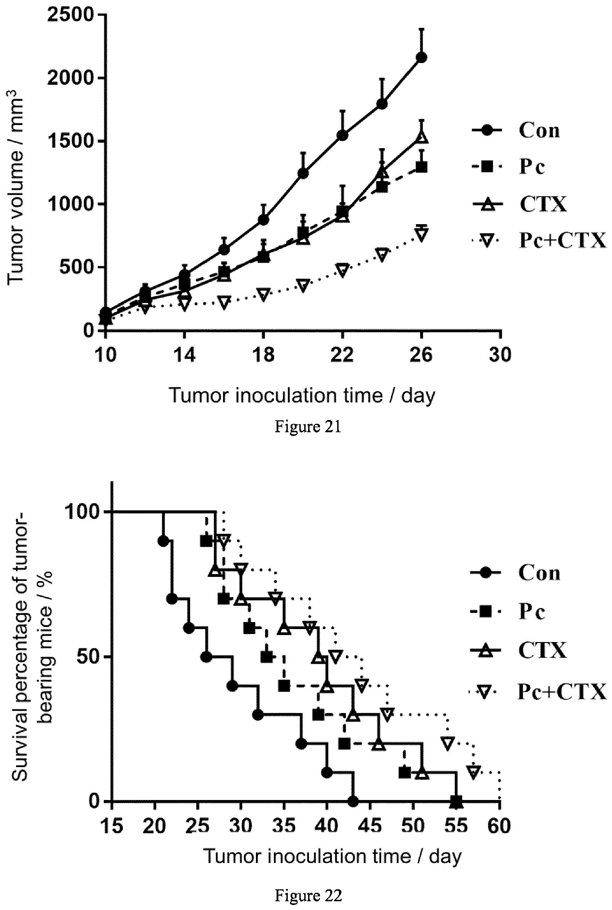

(I) As shown in FIG. 21 and Table 11, the tumor control group (Con), the *Plasmodium* treatment group (Pc), the cyclophosphamide treatment group (CTX) and the treatment group of cyclophosphamide in combination with *Plasmodium* (Pc+CTX) all significantly inhibit the growth of lung cancer. Combined therapy more effectively inhibits tumor growth than single *Plasmodium chabaudi* therapy and single cyclophosphamide therapy. This indicates that a combined pharmaceutical composition of cyclophosphamide and *Plasmodium chabaudi* can more effectively inhibit the growth of lung cancer.

**Table 11**

| Intergroup Comparison | Significance |
|---|---|
| Con vs Pc | ** |
| Con vs CTX | * |
| Con vs Pc+ CTX | ** |
| Pcvs Pc+ CTX | * |
| CTX vs Pc+ CTX | ** |

(II) As shown in FIG. 22 and Table 12, the tumor group is 27.5 days, the *Plasmodium chabaudi* treatment group is 34.5 days, the cyclophosphamide treatment group is 39.5 days, and the combined treatment group is 42.5 days. The combined therapy has the longer median survival time than the single *Plasmodium* therapy and the single cyclophosphamide therapy, but their survival differences have no statistical significance, but still suggests that the combined pharmaceutical composition of cyclophosphamide and *Plasmodium chabaudi* may prolong the survival of tumor-bearing mice better than the single therapy.

**Table 12**

| Intergroup Comparison | Significance |
|---|---|
| Con vs CTX | * |
| Con vs Pc+CTX | ** |

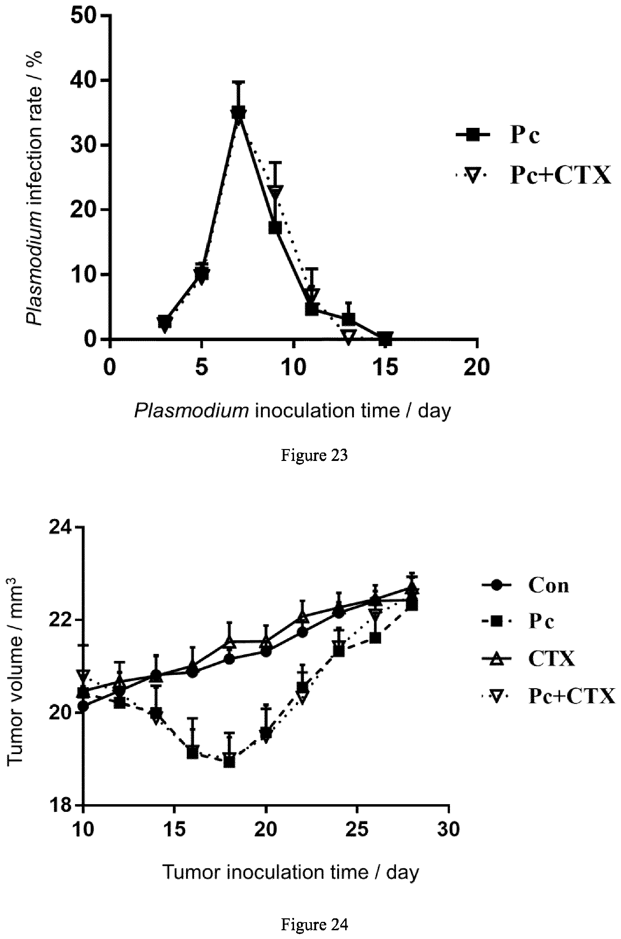

(III) As shown in FIG. 23, tumor-bearing mice in the *Plasmodium chabaudi* treatment group and the combined treatment group have a relatively consistent parasitemia infection duration period, and cyclophosphamide has a relatively small effect on *Plasmodium chabaudi.* This indicates that cyclophosphamide can be combined with *Plasmodium chabaudi* therapy for the treatment of lung cancer, and two therapies have little interference.
(IV) As shown in FIG. 24, the weight of tumor-bearing mice in the combined treatment group is mainly affected by *Plasmodium chabaudi* and affected little by cyclophosphamide, and cyclophosphamide in combination with *Plasmodium* has a slight synergistic effect in reducing the weight of tumor-bearing mice without significantly increasing toxic and side effects on tumor-bearing mice.

**(6) Summary:** The combined therapy of cyclophosphamide and *Plasmodium chabaudi* can more effectively inhibit the growth of lung cancer and prolong the median survival time of tumor-bearing mice than single cyclophosphamide and single *Plasmodium chabaudi* without increasing the toxic and side effects on tumor-bearing mice. Cyclophosphamide administrated in three divided doses on day 6, day 13 and day 20 at a total dosage of 60 mg/kg and *Plasmodium chabaudi* inoculated on day 7 are a potential combined pharmaceutical composition for the treatment of lung cancer.

### Example 7

### This example provides an anti-tumor combined pharmaceutical composition. The combined pharmaceutical composition includes Plasmodium yoelii and a chemotherapeutic agent pemetrexed. The effect of an optimized regimen of pemetrexed administration in combination with Plasmodium yoelii therapy in treating lung cancer was observed.

Experimental materials and reagents required in this example differed from those in Example 3 only in that the chemotherapeutic agent was pemetrexed (abbreviated as PEM), which was purchased from Sigma-Aldrich.

### (1) An animal model was established by the following specific method:

Steps (I), (II) and (III) were the same as those in Example 3.

(IV) Experimental grouping: mice were randomly divided into four groups according to a size of tumor: a tumor control group (Con), a *Plasmodium* treatment group (Py), a pemetrexed treatment group (PEM) and a treatment group of pemetrexed in combination with *Plasmodium* (Py+PEM). There were 10 mice in each group and 40 mice in total.

### (2) Tumor-bearing mice were inoculated with Plasmodium by the same specific method as in Example 3.

### (3) Pemetrexed was administered by the following specific method:

(I) Dode of administration: intraperitoneal injection;
(II) Dosage of administration: 20 mg/kg×6;
(III) Drug preparation: pemetrexed was dissolved in normal saline to be prepared into a solution with a concentration of 2 mg/mL;
(IV) Time of administration: in the PEM group and the Py+PEM group, drug was administered on day 3, day 7, day 10, day 14, day 17 and day 21 after tumor inoculation at a dosage of 20 mg/kg each time.

### (4) Detection indicators were the same as those in Example 3.

### (5) Experimental results

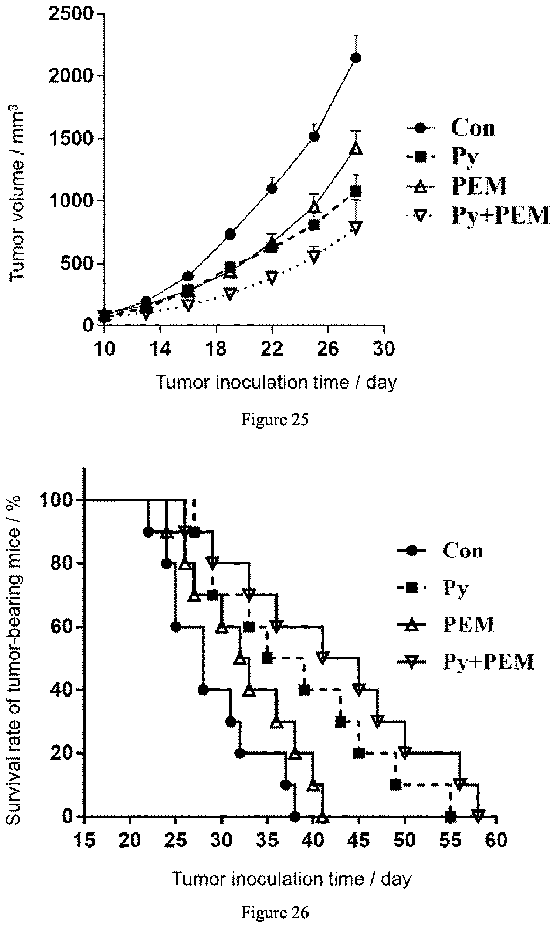

(I) As shown in FIG. 25 and Table 13, the tumor control group (Con), the *Plasmodium* treatment group (Py), the pemetrexed treatment group (PEM) and the treatment group of pemetrexed in combination with *Plasmodium* (Py+PEM) all significantly inhibit the growth of lung cancer. Combined therapy more effectively inhibits tumor growth than single pemetrexed therapy and single *Plasmodium yoelii* therapy. This indicates that a combined pharmaceutical composition of pemetrexed and *Plasmodium yoelii* can more effectively inhibit the growth of lung cancer.

**Table 13**

| Intergroup Comparison | Significance |
|---|---|
| Con vs Py | ** |
| Con vs PEM | ** |
| Con vs Py+PEM | ** |
| Py vs Py+PEM | * |
| PEM vs Py+PEM | ** |

(II) As shown in FIG. 26 and Table 14, the median survival time of the tumor control group is 28 days, the median survival time of the *Plasmodium yoelii* treatment group is 37 days, the median survival time of the pemetrexed treatment group is 32.5 days, and the median survival time of the combined treatment group is 43 days. The combined therapy has the longer median survival time than the single *Plasmodium* therapy and the single *Plasmodium* therapy, a survival difference between the combined therapy and the single pemetrexed therapy has statistical significance, and a survival difference between the combined therapy and the single *Plasmodium* therapy has no statistical significance. This suggests that the combined pharmaceutical composition of pemetrexed and *Plasmodium yoelii* may more effectively prolong the survival time of tumor-bearing mice than the single therapy.

**Table 14**

| Intergroup Comparison | Significance |
|---|---|
| Con vs Py | * |
| Con vs Py+PEM | ** |
| PEM vs Py+PEM | * |

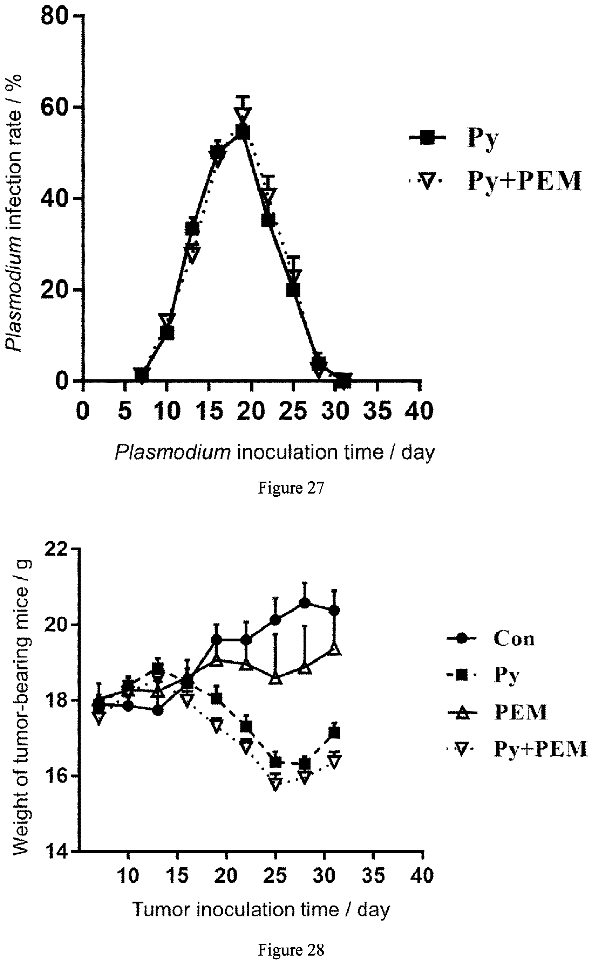

(III) As shown in FIG. 27, tumor-bearing mice in the *Plasmodium yoelii* treatment group and the combined treatment group have a relatively consistent parasitemia duration period. Pemetrexed has a relatively small effect on *Plasmodium.*
(IV) As shown in FIG. 28, the weight of tumor-bearing mice in the combined treatment group is doubly affected by *Plasmodium chabaudi* and pemetrexed, and pemetrexed in combination with *Plasmodium* has a synergistic effect in reducing the weight of tumor-bearing mice and affects the survival of tumor-bearing mice.

**(6) Summary:** The combined therapy of pemetrexed and *Plasmodium yoelii* can more effectively inhibit the growth of lung cancer and prolong the median survival time of tumor-bearing mice. Pemetrexed has no inhibitory effect on *Plasmodium yoelii,* but the combined therapy reduces the weight of tumor-bearing mice and affects the survival of the tumor-bearing mice. Pemetrexed administrated in six divided doses on day 3, day 7, day 10, day 13, day 17 and day 20 at a total dosage of 120 mg/kg and *Plasmodium yoelii* inoculated on day 7 are a potential combined pharmaceutical composition for the treatment of lung cancer.

### Example 8

### This example provides an anti-tumor combined pharmaceutical composition. The combined pharmaceutical composition includes Plasmodium yoelii and a chemotherapeutic agent cis-platinum. The effect of cis-platinum in combination with Plasmodium yoelii therapy in treating lung cancer was observed.

Experimental materials required in this example differed from those in Example 3 only in that the chemotherapeutic agent was cis-platinum (abbreviated as DDP), which was purchased from Sigma-Aldrich.

### (1) An animal model was established by the following specific method:

Steps (I), (II) and (III) were the same as those in Example 3.

(IV) Experimental grouping: mice were randomly divided into four groups according to a size of tumor: a tumor control group (Con), a *Plasmodium* treatment group (Py), a cis-platinum treatment group (DDP) and a treatment group of cis-platinum in combination with *Plasmodium* (Py+DDP). There were 11 mice in each group and 44 mice in total.

### (2) Tumor-bearing mice were inoculated with Plasmodium by the same specific method as in Example 3.

### (3) Cis-platinum was administered by the following specific method:

(I) Mode of administration: intraperitoneal injection;
(II) Dosage of administration: 1 mg/kg×7;
(III) Drug preparation: cis-platinum was dissolved in normal saline to be prepared into a solution with a concentration of 0.1 mg/mL;
(IV) Time of administration: in the DDP treatment group and the Py+DDP treatment group, drug was administered from day 7 after tumor inoculation, where the drug was administered every two days for two weeks, with a dosage of 1 mg/kg each time.

### (4) Detection indicators were the same as those in Example 3.

### (5) Experimental results

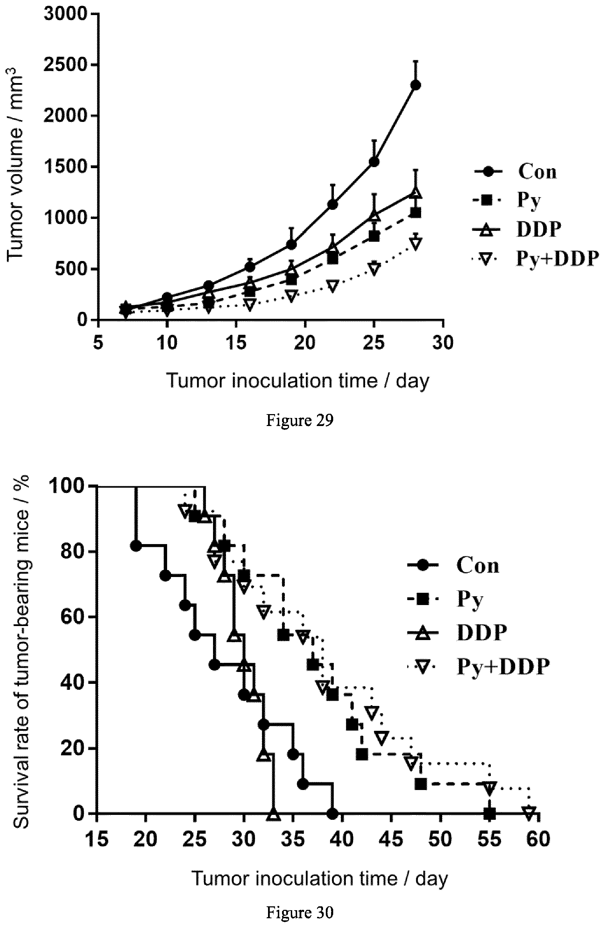

(I) As shown in FIG. 29 and Table 15, the tumor control group (Con), the *Plasmodium* treatment group (Py), the cis-platinum treatment group (DDP) and the treatment group of cis-platinum in combination with *Plasmodium* (Py+DDP) all significantly inhibit the growth of lung cancer. Combined therapy more effectively inhibits tumor growth than single cis-platinum therapy and single *Plasmodium yoelii* therapy. This indicates that a combined pharmaceutical composition of cis-platinum and *Plasmodium yoelii* can more effectively inhibit the growth of lung cancer.

**Table 15**

| Intergroup Comparison | Significance |
|---|---|
| Con vs Py | ** |
| Con vs DDP | ** |
| Con vs Py+DDP | ** |
| Py vs Py+DDP | * |
| DDP vs Py+DDP | * |

(II) As shown in FIG. 30 and Table 16, the median survival time of tumor-bearing mice is compared: the median survival time of the tumor group is 27 days, the median survival time of the *Plasmodium yoelii* treatment group is 37 days, the median survival time of the cis-platinum treatment group is 30 days, and the median survival time of the combined treatment group is 38 days. *Plasmodium* therapy and the combined therapy both prolong the survival of tumor-bearing mice. Compared with the single *Plasmodium* treatment group, the combined treatment group does not significantly prolong the median survival time of tumor-bearing mice, and a survival difference therebetween has no statistical significance.

**Table 16**

| Intergroup Comparison | Significance |
|---|---|
| Con vs Py | * |
| Con vs Py+DDP | * |
| DDP vs Py+DDP | * |

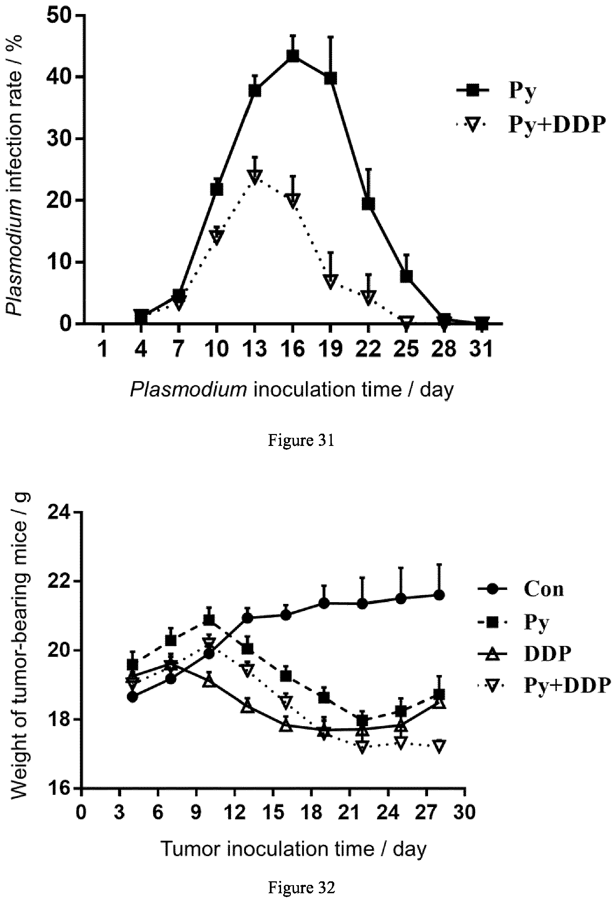

(III) As shown in FIG. 31, tumor-bearing mice in the combined treatment group have a shorter parasitemia duration period and a smaller parasitemia peak value than the single *Plasmodium yoelii* treatment group, suggesting that cis-platinum has an inhibitory effect on *Plasmodium.*
(IV) As shown in FIG. 32, tumor-bearing mice in the treatment group of cis-platinum in combination with *Plasmodium yoelii* are doubly affected by cis-platinum and *Plasmodium,* both of which reduce the weight of the tumor-bearing mice.

**(6) Summary:** The combined therapy of cis-platinum and *Plasmodium yoelii* more effectively inhibits the growth of lung cancer than single cis-platinum therapy and single *Plasmodium yoelii* therapy but does not significantly prolong the median survival time. Cis-platinum has a significant effect on the infection period of *Plasmodium yoelii,* and the combined administration of cis-platinum and *Plasmodium yoelii* reduces the weight of tumor-bearing mice. Cis-platinum administered for two weeks from day 7 at a total dosage of 7 mg/kg and *Plasmodium yoelii* inoculated on day 7 are a potential combined pharmaceutical composition for the treatment of lung cancer.

### Example 9

### This example provides an anti-tumor combined pharmaceutical composition. The combined pharmaceutical composition includes Plasmodium yoelii and mitomycin. The effects of different dosages of mitomycin in combination with Plasmodium yoelii immunotherapy in treating lung cancer were observed.

Experimental materials and reagents required in this example differed from those in Example 3 only in that the chemotherapeutic agent was mitomycin (abbreviated as MMC), which was purchased from Sigma-Aldrich.

### (1) An animal model was established by the following specific method:

Steps (I), (II) and (III) were the same as those in Example 3.

(IV) Experimental grouping: mice were randomly divided into five groups according to a size of tumor: a tumor control group (Con), a *Plasmodium* treatment group (Py), a treatment group of a low dosage of mitomycin in combination with *Plasmodium* (Py+MMC (low dosage)), a treatment group of a medium dosage of mitomycin in combination with *Plasmodium* (Py+MMC (medium dosage)) and a treatment group of a high dosage of mitomycin in combination with *Plasmodium* (Py+MMC (high dosage)). There were 10 mice in each group and 50 mice in total.

### (2) Tumor-bearing mice were inoculated with Plasmodium by the same specific method as in Example 3.

### (3) Mitomycin was administered by the following specific method:

(I) Mode of administration: intraperitoneal injection;
(II) Dosage of administration: a low dosage of 0.027 mg/kg×2, a medium dosage of 0.083 mg/kg×2 and a high dosage of 0.25 mg/kg×2;
(III) Drug preparation: mitomycin was dissolved in normal saline to be prepared into a solution with a concentration of 0.027 mg/mL, a solution with a concentration of 0.083 mg/mL and a solution with a concentration of 0.25 mg/mL;
(IV) Time of administration: in the Py+MMC (low dosage), Py+MMC (medium dosage) and LLC+Py+MMC (high dosage) groups, drug was administered on day 11 and day 18 after tumor inoculation.

### (4) Detection indicators were the same as those in Example 3.

### (5) Experimental results

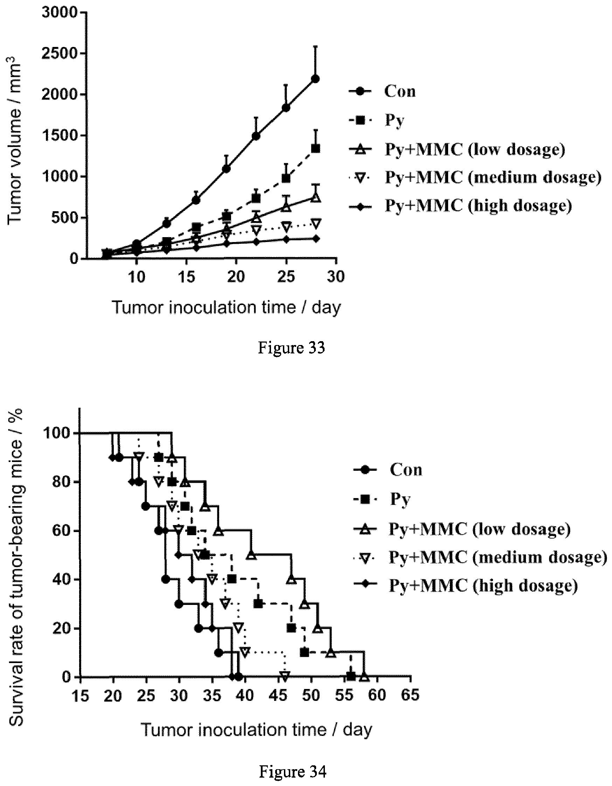

(I) As shown in FIG. 33 and Table 17, the tumor control group (Con), the *Plasmodium* treatment group (Py), the treatment group of the low dosage of mitomycin in combination with *Plasmodium* (Py+MMC (low dosage)), the treatment group of the medium dosage of mitomycin in combination with *Plasmodium* (Py+MMC (medium dosage)) and the treatment group of the high dosage of mitomycin in combination with *Plasmodium* (Py+MMC (high dosage)) all significantly inhibit the growth of lung cancer. Combined therapy exhibits a dosage-dependent effect of mitomycin, and the higher the dosage, the better the growth of lung cancer is inhibited. This indicates that the combined administration of *Plasmodium yoelii* and mitomycin can more effectively inhibit the growth of lung cancer.

**Table 17**

| Intergroup Comparison | Significance |
|---|---|
| Con vs Py | ** |
| Con vs Py+MMC (low dosage) | ** |
| Con vs Py+MMC (medium dosage) | ** |
| Con vs Py+MMC (high dosage) | ** |
| Py vs Py+MMC (low dosage) | * |
| Py vs Py+MMC (medium dosage) | ** |
| Py vs Py+MMC (high dosage) | ** |
| Py+MMC (low dosage) vs Py+MMC (high dosage) | ** |
| Py+MMC (medium dosage) vs Py+MMC (high dosage) | ** |

(II) As shown in FIG. 34 and Table 18, the tumor group is 28 days, the single *Plasmodium yoelii* treatment group is 36 days, the combined treatment group of the low dosage of mitomycin is 44 days, the combined treatment group of the medium dosage of mitomycin is 34 days, and the combined treatment group of the high dosage of mitomycin is 31 days. The combined treatment group of the low dosage of mitomycin more effectively prolongs the survival time of tumor-bearing mice than the combined treatment group of the medium dosage of mitomycin and the combined treatment group of the high dosage of mitomycin, indicating that the low dosage of mitomycin in combination with *Plasmodium yoelii* can more effectively prolong the survival time of tumor-bearing mice. The medium dosage of mitomycin and the high dosage of mitomycin may have certain toxic and side effects.

**Table 18**

| Intergroup Comparison | Significance |
|---|---|
| Con vs Py | * |
| Con vs Py+MMC (low dosage) | ** |
| Py+MMC (low dosage) vs Py+MMC (high dosage) | * |
| Py+MMC (medium dosage) vs Py+MMC (high dosage) | ** |

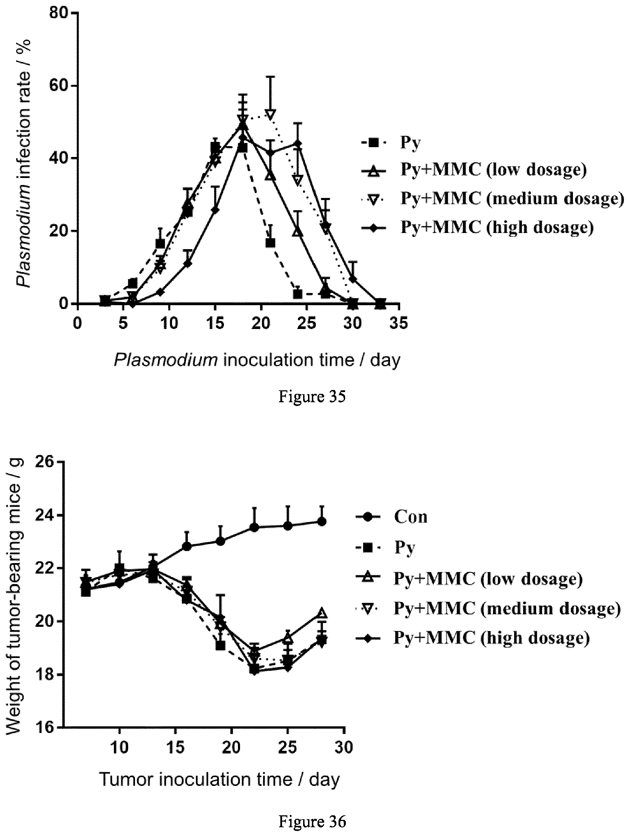

(III) As shown in FIG. 35, a parasitemia peak appears at different times for tumor-bearing mice in the three combined treatment groups, and the higher concentration mitomycin has, the later the peak appears. Mitomycin has a certain inhibitory effect on *Plasmodium* (the higher concentration, the more obvious the inhibitory effect) but will not eliminate *Plasmodium.*
(IV) As shown in FIG. 36, the weight of the tumor-bearing mice in the three combined treatment groups is mainly affected by *Plasmodium* and affected little by mitomycin.

**(6) Summary:** The combined therapy of a low dosage administration of mitomycin and *Plasmodium yoelii* can more effectively inhibit the growth of lung cancer and prolong the survival time of tumor-bearing mice. The low dosage of mitomycin has a slight inhibitory effect on *Plasmodium yoelii* but will not eliminate *Plasmodium.* The medium dosage of mitomycin and the high dosage of mitomycin have certain toxic and side effects and are not suitable for being combined with *Plasmodium* for treating lung cancer. Mitomycin administrated in divided doses on day 11 and day 18 at a total dosage of 0.054 mg/kg and *Plasmodium yoelii* inoculated on day 7 are a potential combined pharmaceutical composition for the treatment of lung cancer.

### Example 10

### This example provides an anti-tumor combined pharmaceutical composition. The combined pharmaceutical composition includes Plasmodium yoelii and a chemotherapeutic agent docetaxel. The effects of different dosages of docetaxel in combination with Plasmodium yoelii immunotherapy in treating lung cancer were observed.

Experimental materials and reagents required in this example differed from those in Example 9 only in that the chemotherapeutic agent was docetaxel (abbreviated as DTX), which was purchased from SANOFI.

### (1) An animal model was established by the following specific method:

Steps (I), (II) and (III) were the same as those in Example 9.

(IV) Experimental grouping: mice were randomly divided into five groups according to a size of tumor: a tumor control group (Con), a *Plasmodium* treatment group (Py), a treatment group of a low dosage of docetaxel in combination with *Plasmodium* (Py+DTX (low dosage)), a treatment group of a medium dosage of docetaxel in combination with *Plasmodium* (Py+MMC (medium dosage)) and a treatment group of a high dosage of docetaxel in combination with *Plasmodium* (Py+MMC (high dosage)). There were 10 mice in each group and 50 mice in total.

### (2) Tumor-bearing mice were inoculated with Plasmodium by the same specific method as in Example 9.

### (3) Docetaxel was administered by the following specific method:

(I) Mode of administration: intravenous injection;
(II) Dosage of administration: a low dosage of 2.2 mg/kg×3, a medium dosage of 6.7 mg/kg×3 and a high dosage of 2 mg/kg×3;
(III) Drug preparation: docetaxel was dissolved in normal saline to be prepared into a solution with a concentration of 0.22 mg/mL, a solution with a concentration of 0.673 mg/mL and a solution with a concentration of 2 mg/mL;
(IV) Time of administration: in the Py+DTX (low dosage), Py+DTX (medium dosage) and Py+DTX (high dosage) groups, drug was administered on day 3, day 10 and day 17 after tumor inoculation.

### (4) Detection indicators were the same as those in Example 9.

### (5) Experimental results

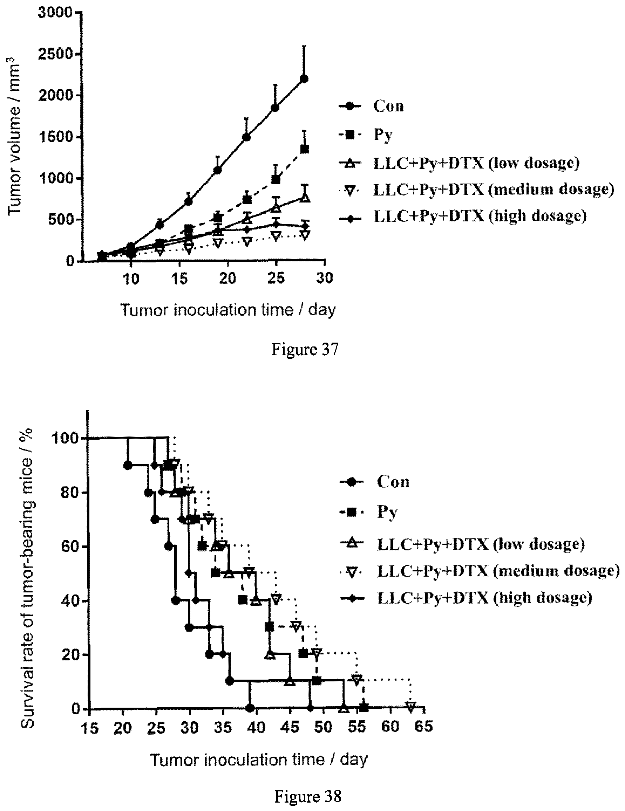

(I) As shown in FIG. 37 and Table 19, the tumor control group (Con), the *Plasmodium* treatment group (Py), the treatment group of the low dosage of docetaxel in combination with *Plasmodium* (Py+DTX (low dosage)), the treatment group of the medium dosage of docetaxel in combination with *Plasmodium* (Py+MMC (medium dosage)) and the treatment group of the high dosage of docetaxel in combination with *Plasmodium* (Py+MMC (high dosage)) can all significantly inhibit the growth of lung cancer. All the three combined treatment groups have better effects than single *Plasmodium* therapy in inhibiting the growth of lung cancer. Among combined therapy, combined therapy of the medium dosage of docetaxel has the best anti-tumor effect, followed by the high dosage with the better effect and the low dosage with the worst effect. This indicates that docetaxel in combination with *Plasmodium yoelii* can more effectively inhibit the growth of lung cancer without exhibiting a dosage-dependence relationship of docetaxel.

**Table 19**

| Intergroup Comparison | Significance |
|---|---|
| Con vs Py | ** |
| Con vs Py+DTX (low dosage) | ** |
| Con vs Py+DTX (medium dosage) | ** |
| Con vs Py+DTX (high dosage) | ** |
| Py vs Py+DTX (low dosage) | * |
| Py vs Py+DTX (medium dosage) | ** |
| Py vs Py+DTX (high dosage) | ** |
| Py+DTX (low dosage) vs Py+DTX (high dosage) | ** |
| Py+DTX (medium dosage) vs Py+DTX (high dosage) | * |

(II) As shown in FIG. 38 and Table 20, the tumor group is 28 days, the single *Plasmodium yoelii* treatment group is 36 days, the combined treatment group of the low dosage of docetaxel is 38 days, the combined treatment group of the medium dosage of docetaxel is 41 days, and the combined treatment group of the high dosage of docetaxel is 30.5 days. The single *Plasmodium yoelii* therapy, combined therapy of the medium dosage of docetaxel and combined therapy of the low dosage of docetaxel can more effectively prolong the survival time of tumor-bearing mice, while combined therapy of the high dosage of docetaxel cannot prolong the survival time of tumor-bearing mice. Survival differences of the combined therapy of the medium dosage and the combined therapy of the low dosage from the single *Plasmodium yoelii* therapy have no statistical significance. The medium dosage of docetaxel and the high dosage of docetaxel may have certain toxic and side effects.

**Table 20**

| Intergroup Comparison | Significance |
|---|---|
| Con vs Py | * |
| Con vs Py+DTX (low dosage) | ** |
| Con vs Py+DTX (medium dosage) | ** |
| Py+DTX (medium dosage) vs Py+DTX (high dosage) | * |

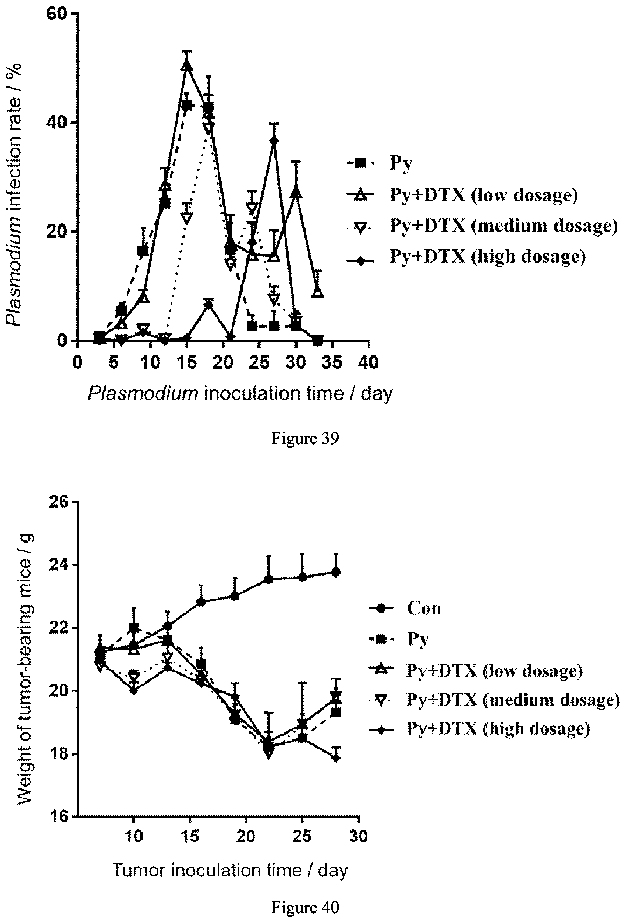

(III) As shown in FIG. 39, tumor-bearing mice in the single *Plasmodium yoelii* treatment group and the three combined treatment groups have inconsistent parasitemia duration periods. This indicates that the medium dosage of docetaxel and the high dosage of docetaxel significantly inhibit *Plasmodium* so that the infection period is abnormal, and the low dosage of docetaxel has a relatively small effect on *Plasmodium.*
(IV) As shown in FIG. 40, the weight of the tumor-bearing mice in the single *Plasmodium yoelii* treatment group and the combined treatment groups are doubly affected by *Plasmodium* and docetaxel. In the combined therapy, the high dosage of docetaxel has a very significant effect in reducing the weight of the tumor-bearing mice, while the medium dosage of docetaxel and the low dosage of docetaxel have relatively small effects.

**(6) Summary:** The combined therapy of a medium dosage of docetaxel and *Plasmodium yoelii* can more effectively inhibit the growth of lung cancer and prolong the median survival time of tumor-bearing mice. Docetaxel administrated in divided doses on day 3, day 10 and day 17 at a total dosage of 6.7 mg/kg and *Plasmodium yoelii* inoculated on day 7 are a potential combined pharmaceutical composition for the treatment of lung cancer.

### Example 11

### This example provides an anti-tumor combined pharmaceutical composition. The combined pharmaceutical composition includes Plasmodium yoelii and a chemotherapeutic agent etoposide. The effects of different dosages of etoposide in combination with Plasmodium yoelii immunotherapy in treating lung cancer were observed.

Experimental materials and reagents required in this example differed from those in Example 9 only in that the chemotherapeutic agent was etoposide (abbreviated as VP16), which was purchased from Bristol Myers Squibb.

### (1) An animal model was established by the following specific method:

Steps (I), (II) and (III) were the same as those in Example 9.

(IV) Experimental grouping: mice were randomly divided into five groups according to a size of tumor: a tumor control group (Con), a *Plasmodium* treatment group (Py), a treatment group of a low dosage of etoposide in combination with *Plasmodium* (Py+VP16 (low dosage)) and a treatment group of a high dosage of docetaxel in combination with *Plasmodium* (Py+VP16 (high dosage)). There were 10 mice in each group and 40 mice in total.

### (2) Tumor-bearing mice were inoculated with Plasmodium by the same specific method as in Example 9.

### (3) Etoposide was administered by the following specific method:

(I) Mode of administration: intraperitoneal injection;
(II) Dosage of administration: a low dosage of 10 mg/kg×3 and a high dosage of 30 mg/kg×3;
(III) Drug preparation: etoposide was dissolved in normal saline to be prepared into a solution with a concentration of 1 mg/mL and a solution with a concentration of 3 mg/mL;
(IV) Time of administration: in the Py+VP16 (low dosage) and Py+VP16 (high dosage) groups, drug was administered on day 11, day 14 and day 17 after tumor inoculation.

### (4) Detection indicators were the same as those in Example 9.

### (5) Experimental results

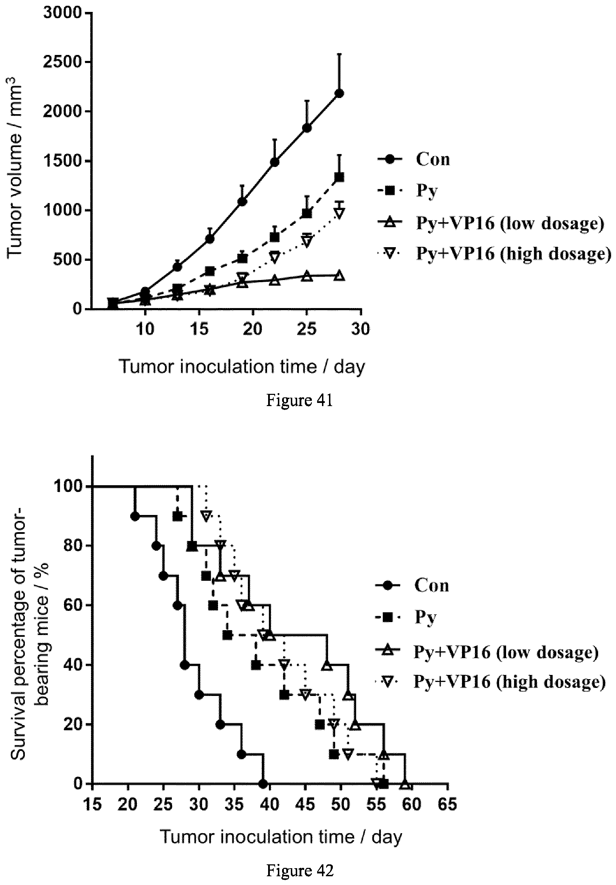

(I) As shown in FIG. 41 and Table 21, the tumor control group (Con), the *Plasmodium* treatment group (Py), the treatment group of the low dosage of etoposide in combination with *Plasmodium* (Py+VP16 (low dosage)) and the treatment group of the high dosage of docetaxel in combination with *Plasmodium* (Py+VP16 (high dosage)) can all significantly inhibit the growth of lung cancer. Combined therapy of the low dosage of etoposide has a better effect than combined therapy of the high dosage of etoposide in inhibiting the growth of lung cancer. The combined therapy of the low dosage of etoposide has a better effect than single *Plasmodium* therapy in inhibiting the growth of lung cancer. This indicates that a combined pharmaceutical composition of a low dosage of etoposide and *Plasmodium yoelii* can more effectively inhibit the growth of lung cancer.

**Table 21**

| Intergroup Comparison | Significance |
|---|---|
| Con vs Py | ** |
| Con vs Py+VP 16 (low dosage) | ∗∗ |
| Con vs Py+VP 16 (high dosage) | ∗∗∗ |
| Py vs Py+VP 16 (low dosage) | ∗ |
| Py+VP 16 (low dosage) vs Py+VP16 (high dosage) | ∗ |

(II) As shown in FIG. 42 and Table 22, the tumor group is 28 days, the *Plasmodium yoelii* treatment group is 36 days, the combined treatment group of the low dosage of etoposide is 44 days, and the combined treatment group of the high dosage of etoposide is 40.5 days. Single *Plasmodium yoelii* therapy, the combined therapy of the high dosage of etoposide and the combined therapy of the low dosage of etoposide can all prolong the survival time of tumor-bearing mice. The combined therapy of the low dosage of etoposide has a longer median survival time than the combined therapy of the high dosage of etoposide, but a survival difference therebetween has no statistical significance. The high dosage of etoposide may have certain toxic and side effects. The combined therapy of the low dosage of etoposide and *Plasmodium yoelii* may effectively prolong the survival time of tumor-bearing mice.

**Table 22**

| Intergroup Comparison | Significance |
|---|---|
| Con vs Py | ∗ |
| Con vs Py+VP 16 (low dosage) | ∗∗ |
| Con vs Py+VP 16 (high dosage) | ∗∗ |

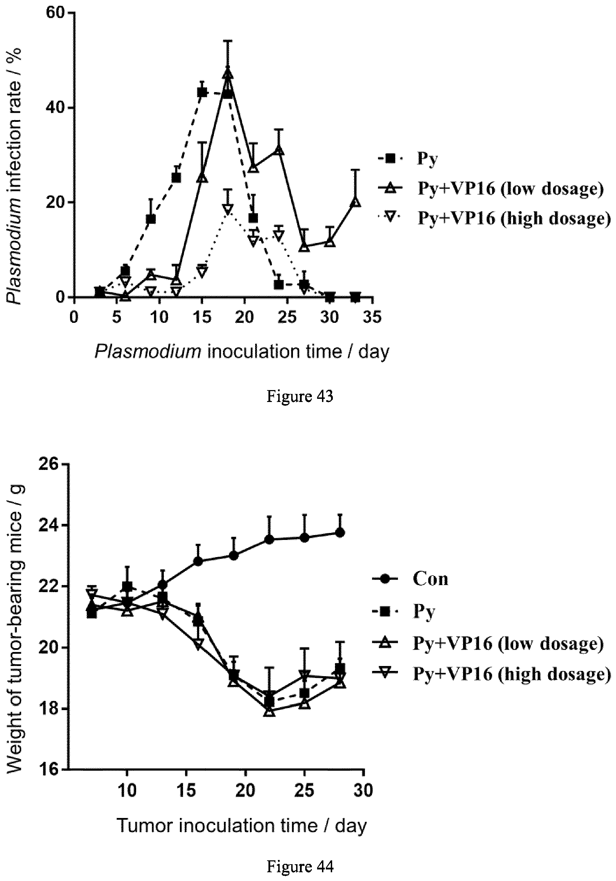

(III) As shown in FIG. 43, tumor-bearing mice in the *Plasmodium yoelii* treatment group and two treatment groups in combination with *Plasmodium yoelii* have an inconsistent parasitemia duration period. This indicates that etoposide has an obvious inhibitory effect on *Plasmodium.*
(IV) As shown in FIG. 44, the weight of tumor-bearing mice in the treatment groups of the high dosage of etoposide and the low dosage of etoposide in combination with *Plasmodium yoelii* is doubly affected by *Plasmodium* and etoposide, which further reduces the weight of the tumor-bearing mice.

**(6) Summary:** The low dosage of etoposide in combination with *Plasmodium yoelii* can more effectively inhibit the growth of lung cancer and prolong the median survival time of tumor-bearing mice. The high dosage of etoposide and the low dosage of etoposide both have inhibitory effects on *Plasmodium yoelii* infection but will not eliminate *Plasmodium.* Etoposide administrated in divided doses on day 11, day 14 and day 17 at a total dosage of 30 mg/kg and *Plasmodium yoelii* inoculated on day 7 are a potential combined pharmaceutical composition for the treatment of lung cancer.

### Example 12

### This example provides an anti-tumor combined pharmaceutical composition. The combined pharmaceutical composition includes Plasmodium yoelii and an HIV protease inhibitor nelfinavir. The effect of nelfinavir administration in combination with Plasmodium yoelii immunotherapy in treating lung cancer was observed.

Experimental materials and reagents required in this example differed from those in Example 9 only in that the chemotherapeutic agent was nelfinavir (abbreviated as NFV), which was purchased from Agouron Pharmaceuticals, Inc.

### (1) An animal model was established by the following specific method:

Steps (I), (II) and (III) were the same as those in Example 9.

(IV) Experimental grouping: mice were randomly divided into four groups according to a size of tumor: a tumor control group (Con), a *Plasmodium* treatment group (Py), a nelfinavir treatment group (NFV) and a treatment group of nelfinavir in combination with *Plasmodium* (Py+CTX). There were 10 mice in each group and 40 mice in total.

### (2) Tumor-bearing mice were inoculated with Plasmodium by the same specific method as in Example 9.

### (3) Nelfinavir was administered by the following specific method:

(I) Mode of administration: intraperitoneal injection;
(II) Dosage of administration: 400 mg/kg× 10;
(III) Drug preparation: nelfinavir was dissolved in normal saline to be prepared into a solution with a concentration of 40 mg/mL;
(IV) Time of administration: in the NFV group and the Py+NFV group, drug was administered from day 10 after tumor inoculation for 10 days at a dosage of 400 mg/kg each time.

### (4) Detection indicators were the same as those in Example 9.

### (5) Experimental results

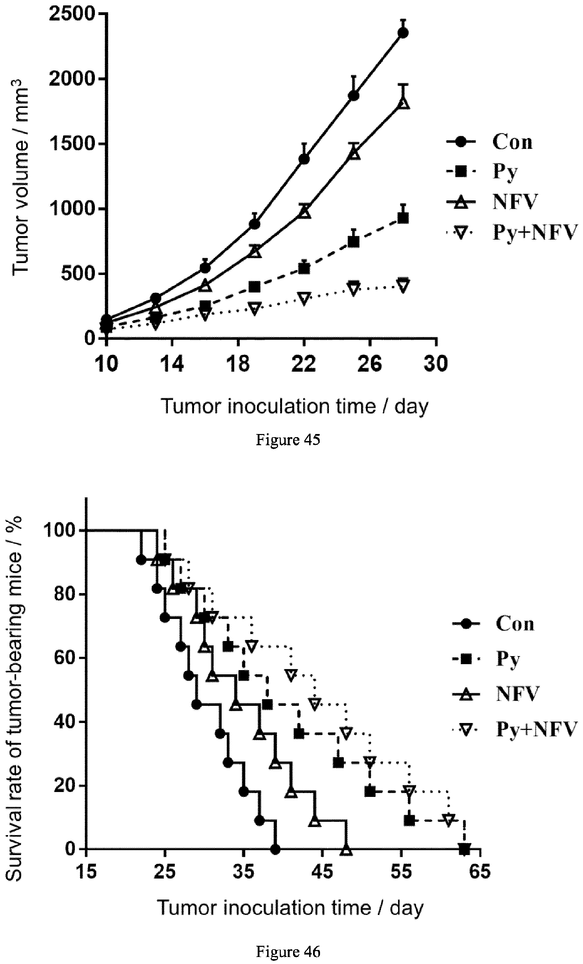

(I) As shown in FIG. 45 and Table 23, the tumor control group (Con), the *Plasmodium* treatment group (Py), the nelfinavir treatment group (NFV) and the treatment group of nelfinavir in combination with *Plasmodium* (Py+CTX) all effectively inhibit the growth of lung cancer. Combined therapy can more effectively inhibit tumor growth than single nelfinavir therapy and single *Plasmodium yoelii.* This indicates that a combined pharmaceutical composition of nelfinavir and *Plasmodium yoelii* can more effectively inhibit the growth of lung cancer.

**Table 23**

| Intergroup Comparison | Significance |
|---|---|
| Con vs Py | ** |
| Con vs NFV | ** |
| Con vs Py+NFV | ** |
| Py vs Py+NFV | ** |
| NFV vs Py+NFV | ** |

(II) As shown in FIG. 46 and Table 24, the median survival time of tumor-bearing mice is compared: the median survival time of the tumor group is 29 days, the median survival time of the *Plasmodium yoelii* treatment group is 38 days, the median survival time of the nelfinavir treatment group is 34 days, and the median survival time of the treatment group of nelfinavir in combination with *Plasmodium yoelii* is 44 days. The single *Plasmodium yoelii* treatment group and the combined treatment group both more effectively prolong the median survival time of tumor-bearing mice. Combined therapy more effectively prolongs the median survival time of tumor-bearing mice than single nelfinavir therapy, but a survival difference between the combined therapy and single *Plasmodium* therapy has no statistical significance. This suggests that the combined pharmaceutical composition of nelfinavir and *Plasmodium yoelii* may more effectively prolong the median survival time of tumor-bearing mice.

**Table 24**

| Intergroup Comparison | Significance |
|---|---|
| Con vs Py | * |
| Con vs Py+NFV | ** |
| NFV vs Py+NFV | * |

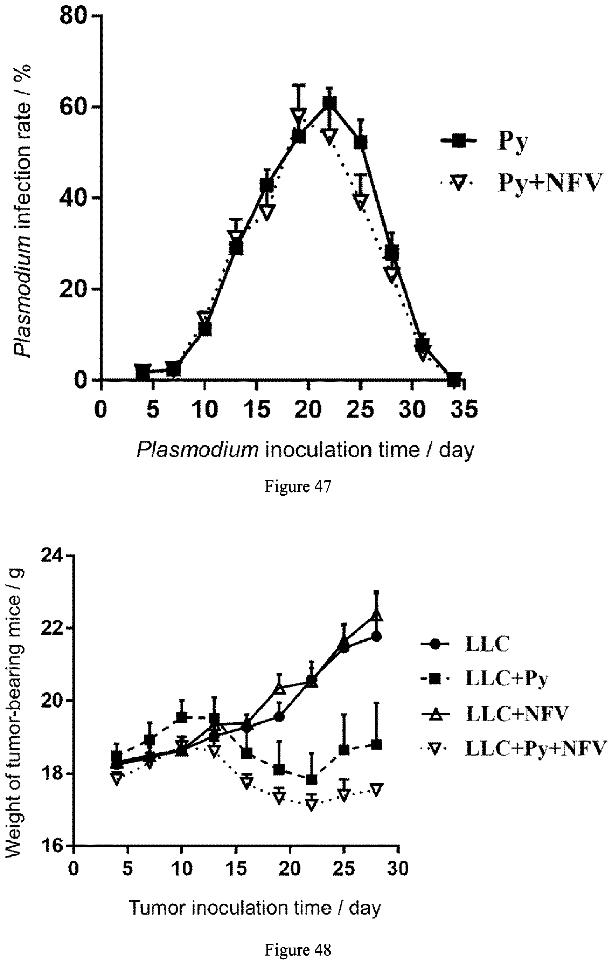

(III) As shown in FIG. 47, tumor-bearing mice in the single *Plasmodium yoelii* treatment group and the combined treatment group have a relatively consistent parasitemia duration period. Nelfinavir has a relatively small effect on *Plasmodium* and can be combined with *Plasmodium yoelii* therapy for the treatment of lung cancer.
(IV) As shown in FIG. 48, the continuous intragastric administration of nelfinavir alone has a relatively small effect on the weight of tumor-bearing mice, but the weight of tumor-bearing mice in the combined treatment group is mainly affected by *Plasmodium* and the continuous intragastric administration of nelfinavir.

**(6) Summary:** The combined therapy of nelfinavir and *Plasmodium yoelii* can more effectively inhibit the growth of lung cancer and prolong the median survival time of tumor-bearing mice. Nelfinavir will not increase toxic and side effects on tumor-bearing mice. Nelfinavir administrated for 10 days from day 10 at a total dosage of 4 g/kg and *Plasmodium yoelii* inoculated on day 7 are a potential combined pharmaceutical composition for the treatment of lung cancer.

The Applicant has stated that multiple combination methods of *Plasmodium* immunotherapy in combination with chemotherapy in the present application and their uses for treating cancer are described through the preceding examples, but the present application is not limited to the preceding examples, which means that the implementation of the present application does not necessarily depend on the preceding examples. It should be apparent to those skilled in the art that any improvements made to the present application, equivalent replacements of raw materials of the product of the present application, additions of adjuvant ingredients to the product of the present application, and selections of specific manners, etc., all fall within the protection scope and the disclosed scope of the present application.

Although the preferred embodiments of the present application have been described above in detail, the present application is not limited to details of the above-described embodiments, and various simple modifications can be made to the technical solutions of the present application without departing from the technical concept of the present application. These simple modifications are all within the protection scope of the present application.

In addition, it is to be noted that if not in collision, the specific technical features described in the preceding embodiments may be combined in any suitable manner. In order to avoid unnecessary repetition, the present application does not further specify any of various possible combination manners.

## Claims

1. An anti-tumor combined pharmaceutical composition, comprising *Plasmodium* and a chemotherapeutic agent.

2. The combined pharmaceutical composition according to claim 1, wherein the *Plasmodium* comprises any one or a combination of at least two of *Plasmodium falciparum, Plasmodium vivax, Plasmodium malariae, Plasmodium ovale* or *Plasmodium knowlesi,* preferably *Plasmodium falciparum* or *Plasmodium vivax.*

3. The combined pharmaceutical composition according to claim 1 or 2, wherein the chemotherapeutic agent comprises an alkylating agent-based chemotherapeutic agent, an anti-metabolic chemotherapeutic agent, an antibiotic-based chemotherapeutic agent, an animal or plant chemotherapeutic agent, a miscellaneous chemotherapeutic agent or an HIV protease inhibitor.

4. The combined pharmaceutical composition according to claim 3, wherein the alkylating agent-based chemotherapeutic agent comprises cyclophosphamide or ifosfamide;
preferably, the anti-metabolic chemotherapeutic agent comprises gemcitabine, pemetrexed, 5-fluorouracil, cytarabine or methotrexate;
preferably, the antibiotic-based chemotherapeutic agent comprises mitomycin, doxorubicin or actinomycin D;
preferably, the animal or plant chemotherapeutic agent comprises etoposide, docetaxel, paclitaxel, vincristine or irinotecan;
preferably, the miscellaneous chemotherapeutic agent comprises cis-platinum, carboplatin, oxaliplatin or asparaginase;
preferably, the HIV protease inhibitor comprises nelfinavir, saquinavir, indinavir or ritonavir.

5. The combined pharmaceutical composition according to claim 1, comprising *Plasmodium* and gemcitabine; *Plasmodium* and cyclophosphamide; *Plasmodium* and cyclophosphamide; *Plasmodium* and pemetrexed; *Plasmodium* and cis-platinum; *Plasmodium* and mitomycin; *Plasmodium* and docetaxel; *Plasmodium* and etoposide; or *Plasmodium* and nelfinavir.

6. The combined pharmaceutical composition according to any one of claims 1 to 5, wherein the combined pharmaceutical composition is in a dosage form that comprises any pharmaceutically acceptable dosage form.

7. The combined pharmaceutical composition according to any one of claims 1 to 6, further comprising any one or a combination of at least two of pharmaceutically acceptable adjuvants.

8. The combined pharmaceutical composition according to any one of claims 1 to 7, wherein the combined pharmaceutical composition is a single compound preparation.

9. The combined pharmaceutical composition according to any one of claims 1 to 7, wherein the combined pharmaceutical composition is a combination of a separate *Plasmodium* preparation and a separate preparation of the chemotherapeutic agent.

10. The combined pharmaceutical composition according to claim 9, wherein the separate *Plasmodium* preparation and the separate preparation of the chemotherapeutic agent are administered simultaneously or sequentially.

11. The combined pharmaceutical composition according to any one of claims 1 to 10, wherein the combined pharmaceutical composition is administered by a route that comprises intravenous injection, intraperitoneal injection, intramuscular injection, subcutaneous injection, oral administration, sublingual administration, nasal administration or percutaneous administration, preferably intravenous injection or oral administration.

12. The combined pharmaceutical composition according to any one of claims 1 to 11, wherein the combined pharmaceutical composition is loaded on a pharmaceutical carrier.

13. The combined pharmaceutical composition according to claim 12, wherein the pharmaceutical carrier comprises a liposome, a micelle, a dendrimer, a microsphere or a microcapsule.

14. Use of the combined pharmaceutical composition according to any one of claims 1 to 13 for preparing a medicament against tumor.

15. The use according to claim 14, wherein tumor comprises lung cancer, gastric cancer, colon cancer, liver cancer, breast cancer or pancreatic cancer.
